(19) Europäisches Patentamt
European Patent Office
Office européen des brevets

(11) **EP 4 274 928 B1**

(12) **EUROPÄISCHE PATENTSCHRIFT**

(45) Veröffentlichungstag und Bekanntmachung des Hinweises auf die Patenterteilung:
**08.01.2025 Patentblatt 2025/02**

(21) Anmeldenummer: **21805396.5**

(22) Anmeldetag: **26.10.2021**

(51) Internationale Patentklassifikation (IPC):
*D04H 1/728* (2012.01)    *D04H 1/4382* (2012.01)
*D04H 1/4209* (2012.01)    *D01D 5/00* (2006.01)
*C12N 5/00* (2006.01)    *A61L 27/56* (2006.01)
*D01F 1/10* (2006.01)    *D01F 1/08* (2006.01)

(52) Gemeinsame Patentklassifikation (CPC):
**D04H 1/728; A61L 27/56; C12N 5/0068; D01D 5/0015; D01F 1/10; D04H 1/4209; D04H 1/43838;** A61L 2400/12; C12N 2533/30; D01F 1/08

(86) Internationale Anmeldenummer:
**PCT/EP2021/079619**

(87) Internationale Veröffentlichungsnummer:
**WO 2022/148564 (14.07.2022 Gazette 2022/28)**

(54) **HOCHPORÖSE NANOFASERVLIESE ALS TRÄGERSTRUKTUR FÜR STROMALES GEWEBE**

HIGH-POROSITY NANOFIBER NONWOVENS AS A SUPPORT STRUCTURE FOR STROMAL TISSUE

NON-TISSÉS DE NANOFIBRES À POROSITÉ ÉLEVÉE EN TANT QUE STRUCTURE DE SUPPORT POUR TISSU STROMALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.01.2021 DE 102021200128**
**01.03.2021 DE 102021201935**

(43) Veröffentlichungstag der Anmeldung:
**15.11.2023 Patentblatt 2023/46**

(73) Patentinhaber: **Fraunhofer-Gesellschaft zur Förderung**
**der angewandten Forschung e.V.**
**80686 München (DE)**

(72) Erfinder:
- **WEIGEL, Tobias**
  **97082 Würzburg (DE)**
- **GROEBER-BECKER, Florian**
  **97082 Würzburg (DE)**
- **HANSMANN, Jan**
  **97082 Würzburg (DE)**
- **CHRIST, Bastian**
  **97082 Würzburg (DE)**
- **PROBST, Jörn**
  **97082 Würzburg (DE)**

(74) Vertreter: **Schrell, Andreas et al**
**Gleiss Große Schrell und Partner mbB**
**Patentanwälte Rechtsanwälte**
**Leitzstrasse 45**
**70469 Stuttgart (DE)**

(56) Entgegenhaltungen:
WO-A1-2007/125288    WO-A1-2011/143213
WO-A1-2015/079278    WO-A1-2016/105581
WO-A2-2013/023064    CN-A- 105 727 364
CN-A- 105 727 364

- JINGLEI WU ET AL: "Enhancing cell infiltration of electrospun fibrous scaffolds in tissue regeneration", BIOACTIVE MATERIALS, vol. 1, no. 1, 26 July 2016 (2016-07-26), pages 56 - 64, XP55465788, ISSN: 2452-199X, DOI: 10.1016/ j.bioactmat.2016.07.001

• SEMITELA ÂNGELA ET AL: "Electrospinning of bioactive polycaprolactone-gelatin nanofibres with increased pore size for cartilage tissue engineering applications", JOURNAL OF BIOMATERIALS APPLICATIONS., vol. 35, no. 4-5, 1 October 2020 (2020-10-01), US, pages 471 - 484, XP093064556, ISSN: 0885-3282, Retrieved from the Internet <URL:https://ria.ua.pt/bitstream/10773/30503/1/Author%20Manuscript%20-%20Electrospinning%20of%20bioactive%20polycaprolactone-gelatin%20nanofibres%20with%20increased%20pore%20size%20for%20cartilage%20tissue%20engineering%20applications.pdf> DOI: 10.1177/0885328220940194
• JINGLEI WU ET AL: "Enhancing cell infiltration of electrospun fibrous scaffolds in tissue regeneration", BIOACTIVE MATERIALS, vol. 1, no. 1, 26 July 2016 (2016-07-26), pages 56 - 64, XP055465788, ISSN: 2452-199X, DOI: 10.1016/j.bioactmat.2016.07.001

• KIM T G ET AL: "Macroporous and nanofibrous hyaluronic acid/collagen hybrid scaffold fabricated by concurrent electrospinning and deposition/leaching of salt particles", ACTA BIOMATERIALIA, ELSEVIER, AMSTERDAM, NL, vol. 4, no. 6, 1 November 2008 (2008-11-01), pages 1611 - 1619, XP025535822, ISSN: 1742-7061, [retrieved on 20080701], DOI: 10.1016/J.ACTBIO.2008.06.008

**Beschreibung**

Die vorliegende Erfindung betrifft Nanofaservliese umfassend ein Netzwerk von aus mindestens einem Nanofasermaterial aufgebauten, Poren umschließenden Nanofasern, Verfahren zur Herstellung von Nanofaservliesen, ihre Verwendung sowie künstliche Gewebe mit diesen Nanofaservliesen und Verfahren zur Herstellung dieser künstlichen Gewebe.

Das Erforschen der komplexen Abläufe in biologischen Geweben stellt die Grundlage für die regenerative Medizin dar. Historisch erfolgten viele dieser Experimente an Tiermodellen, um zum Beispiel Heilungsprozesse, Implantate, Medikamente oder das Verhalten von Tumoren zu untersuchen.

Durch die Unterschiede zwischen Tier und Mensch können aber viele Abläufe nicht korrekt dargestellt werden. Des Weiteren wird derzeit versucht, Tierversuche nach dem 3R-Prinzip (Refine, Reduce, Replace) weitestgehend zu vermeiden. Alternativen bieten *in vitro* generierte Gewebe-Modelle, welche über die Anwendung von humanen Zellen einen Teil des zu untersuchenden Gewebes darstellen.

Für den Aufbau dieser Gewebe werden neben Zellen auch geeignete Trägerstrukturen benötigt, welche die Grundstruktur und Funktion des extrazellulären Gewebes nachbilden. Diese Trägerstrukturen haben dabei entweder einen biologischen oder synthetischen Ursprung. Biologische Trägerstrukturen wie Kollagengele oder dezellularisierte Organe kommen häufig zum Einsatz mit dem Ziel, durch die natürlichen Matrix-Proteine eine möglichst physiologische Umgebung für die Zellen schaffen. Nachteilig ist aber, dass es sich dabei um Material tierischen Ursprungs handelt. In diesen Verfahren sind sowohl Tötungen von Tieren für die Organentnahme, als auch hohe Qualitätsschwankungen zwischen verschiedenen Chargen oder sogar innerhalb einer Charge unvermeidlich.

Ferner kann damit kein vollständig-humanes Gewebemodell erzeugt werden. Daher erfolgen seit vielen Jahren Entwicklungen, die Zellträgerstrukturen über synthetische Wege herzustellen. Trägerstrukturen für Weichgewebe werden dabei hauptsächlich über additive Fertigungstechniken erzeugt, wie zum Beispiel der 3D-Druck von Hydrogelen oder einer Vielzahl an Spinnverfahren.

Das Drucken von Hydrogelen hat den Vorteil, dass in einem Konstrukt verschiedene Zellen und verschiedene Gewebe gleichzeitig verarbeitet werden können. Nachteilig sind aber zum Beispiel die mechanischen Eigenschaften, welche die Anwendbarkeit des Produkts stark begrenzen. Spinnverfahren sind heute in der Lage, fast jeden denkbaren Faserdurchmesser zu erzeugen. Herausfordernd bei den hier relevanten Nanofasern ist aber die Kontrolle über die Faserstruktur. Beispielsweise kann die Orientierung der Fasern einfach eingestellt werden, während aber die Möglichkeiten der Porosität und vor allem die Dimensionen der Porosität für dreidimensionale biologische Gewebeanwendungen sehr limitiert sind. Eine geeignete Porosität ist jedoch für eine ausreichende Integration von Zelle notwendig. Ist die Porosität zu gering, können die Zellen nur in geringer Stückzahl oder gar nicht in das Gewebe eindringen. Bei der Erzeugung von Nanofaservliesen über Elektrospinnen sind die Maschen und Poren bereits so klein, dass Zellen entweder gar nicht oder nur sehr eingeschränkt das Gewebe besiedeln können.

Daher wird nach Lösungen gesucht, die exzellenten mechanischen Eigenschaften von Nanofasern und hohe Verfügbarkeit der verschiedensten Materialien mit Möglichkeiten der 3D-Strukturierung zu kombinieren. Das Ziel ist es, neue Verfahren zur Herstellung nanofaserbasierter 3D-Strukturen für die Erzeugung von 3D-Gewebemodellen zu entwickeln.

Zur Erhöhung der Porosität werden gewöhnlich lösliche Fremdkörper (Porogene) zwischen die Fasern während des Spinnprozesses immobilisiert und anschließend wieder herausgelöst. Die Anwendung von Porogenen mit ähnlichen Dimensionen wie die Fasern der Trägerstruktur (500 nm bis 10000 nm) führt zu einer Lockerung der Faserstruktur und erhöht dabei hauptsächlich die Maschenweite des Faservlieses.

Die Literatur beschreibt dabei eine erhebliche Verbesserung zum Einwandern von Zellen und lässt damit auch eine dreidimensionale Zellkultur zu, es bleibt aber zu wenig Platz, damit die Zellen physiologisch vergleichbares biologisches Gewebe aufbauen können.

In der WO2016/105581 A1 erfolgt die Besiedlung allein über die Poren (interkonnektiert), welche aber kaum flexibel sind und mit Zellen komplett ausgefüllt werden müssen (inhomogene Verteilung zwischen Zellen und Fasern). Gemäß dieser Offenbarung werden die Nanofaservliese gemahlen, um kurze Faserstücke zu erreichen.

In der WO 2007/125288 A1 besitzt das eingesetzte Material dichte Porenwände. Würde man dabei die Porosität erhöhen, funktioniert das ausschließlich über die Porengröße. Die Erhöhung der Porengröße führt zu einem geringeren Oberflächenkrümmen und reduziert dadurch den 3D-Charakter der Membran für die Zellen. Das heißt, größere Poren stellen für die Zellen eine eher 2D-Oberfläche dar.

Das der vorliegenden Erfindung zugrundeliegende technische Problem ist es Nanofaservliese bereitzustellen, welche die vorgenannten Nachteile bekannter Zellträgerstrukturen nicht aufweisen, insbesondere solche, welche es ermöglichen, künstliche Gewebe schneller herstellen zu können, welche, insbesondere bezüglich des Kontaktes der Zellen zueinander, einem natürlichen Gewebe ähnlicher sind als bekannte künstliche Gewebe.

Dieses technische Problem wird gelöst durch ein Nanofaservlies gemäß Anspruch 1, umfassend ein Netzwerk von aus mindestens einem Nanofasermaterial aufgebauten, Poren umschließenden Nanofasern, hergestellt durch ein erstes Verfahren umfassend die Verfahrensschritte:

a) Bereitstellen mindestens eines Nanofasermaterials und mindestens eines zur Ausbildung mindestens eines Porogens geeigneten Porogenmaterials,

b) Elektroverspinnen des mindestens einen Nanofasermaterials unter Einbringen von aus dem mindestens einem Porogenmaterial ausgebildeten mindestens einem Porogen mit einem Durchmesser von 30 bis 1000 µm, so dass das Volumenverhältnis von Porogenmaterial zu Nanofasermaterial in dem nach Abschluss dieses Verfahrensschrittes erhaltenen elektroversponnenen Nanofaservlies 40 bis 99 zu 60 bis 1 (jeweils Volumen, bezogen auf das Gesamtvolumen, also Fasern und Porogene, des Nanofaservlieses) beträgt und

c) Erhalten eines Nanofaservlieses umfassend mindestens ein Porogen.

Das technische Problem wird auch durch das Verfahren zur Herstellung eines Nanofaservlieses gemäß Anspruch 9 gelöst. Insbesondere wird das technische Problem gelöst durch ein erstes Verfahren zur Herstellung eines Nanofaservlieses umfassend ein Netzwerk von aus mindestens einem Nanofasermaterial aufgebauten, Poren umschließenden Nanofasern umfassend die Verfahrensschritte:

a) Bereitstellen mindestens eines Nanofasermaterials und mindestens eines zur Ausbildung mindestens eines Porogens geeigneten Porogenmaterials,

b) Elektroverspinnen des mindestens einen Nanofasermaterials unter Einbringen von aus dem mindestens einem Porogenmaterial ausgebildeten mindestens einem Porogen mit einem Durchmesser von 30 bis 1000 µm, so dass das Volumenverhältnis von Porogenmaterial zu Nanofasermaterial in dem nach Abschluss dieses Verfahrensschrittes erhaltenen elektroversponnenen Nanofaservlies 40 bis 99 zu 60 bis 1 (jeweils Volumen, bezogen auf das Gesamtvolumen, also Fasern und Porogene, des Nanofaservlieses) beträgt und

c) Erhalten eines Nanofaservlieses umfassend mindestens ein Porogen.

In besonders bevorzugter Ausführungsform weist das gemäß eines ersten Verfahrens erhaltene Nanofaservlies eine Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses) auf.

In einer bevorzugten Ausführungsform beträgt das Volumenverhältnis von Porogenmaterial zu Nanofasermaterial in dem nach Abschluss des Verfahrensschrittes b) gemäß des ersten Verfahrens erhaltenen elektroversponnenen Nanofaservlies 50 bis 99 zu 50 bis 1, insbesondere 60 bis 99 zu 40 bis 1, insbesondere 70 bis 99 zu 30 bis 1, insbesondere 80 bis 99 zu 20 bis 1 (jeweils Volumen, bezogen auf das Gesamtvolumen, also Fasern und Porogene, des Nanofaservlieses).

Die Erfindung betrifft auch ein zweites Verfahren zur Herstellung eines erfindungsgemäßen Nanofaservlieses, umfassend folgende Verfahrensschritte:

a') Bereitstellen mindestens eines Nanofasermaterials und mindestens eines zur Ausbildung mindestens eines Porogens geeigneten Porogenmaterials,

b') Elektroverspinnen des mindestens einen Nanofasermaterials unter Einbringen von aus dem mindestens einem Porogenmaterial ausgebildeten mindestens einem Porogen mit einem Durchmesser von 30 bis 1000 µm, so dass das Verhältnis von Porogenmaterial zu Nanofasermaterial in dem nach Abschluss dieses Verfahrensschrittes erhaltenen elektroversponnenen Nanofaservlies 0,07 bis 99,997 zu 99,93 bis 0,003 (jeweils Masse, bezogen auf die Gesamtmasse, also Porogene und Fasern, des Nanofaservlieses) beträgt und

c') Erhalten eines Nanofaservlieses umfassend mindestens ein Porogen, insbesondere eines Nanofaservlieses mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses).

In besonders bevorzugter Ausführungsform weist das gemäß des zweiten Verfahrens erhaltene Nanofaservlies eine Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses) auf.

In einer bevorzugten Ausführungsform beträgt das Verhältnis von Porogenmaterial zu Nanofasermaterial in dem nach Abschluss des Verfahrensschrittes b') gemäß des zweiten Verfahrens erhaltenen elektroversponnenen Nanofaservlies 0,5 bis 99,99 zu 99,5 bis 0,01, insbesondere 1 bis 99,95 zu 99 bis 0,05 , insbesondere 10 bis 99 zu 90 bis 1, insbesondere 20 bis 99 zu 80 bis 1, insbesondere 40 bis 99 zu 60 bis 1, insbesondere 80 bis 99 zu 20 bis 1 (jeweils Masse, bezogen auf die Gesamtmasse, also Fasern und Porogene, des Nanofaservlieses).

Die Erfindung betrifft auch mittels der vorgenannten Verfahren, insbesondere des ersten und zweiten Verfahrens hergestellte Nanofaservliese umfassend ein Netzwerk von aus mindestens einem Nanofasermaterial aufgebauten porenumschließenden Nanofasern.

Ohne an die Theorie gebunden zu sein, bietet das erfindungsgemäße Nanofaservlies insbesondere die Vorteile, dass Zellen (zum Beispiel Fibroblasten) das hochporöse erfindungsgemäße Faservlies zusammenziehen (kontrahieren) können, wie es beispielsweise von Kollagengelen bekannt ist und dass Zellen ihre eigene dreidimensionale Proteinstruktur, insbesondere extrazelluläre Matrix, innerhalb der dreidimensionalen Faserstruktur generieren. Je nach Zelltyp ist diese extrazelluläre Matrix auch spezifisch wie in natürlichem Gewebe. So erzeugen Fibroblasten zum Beispiel Kollagen I innerhalb des Vlieses (wie Bindegewebe), mesenchymale Stromazellen, die adipogen differenziert sind, erzeugen Kollagen IV und an der Grenzfläche zum Epithel wird Laminin und Kollagen IV erzeugt. Das erfindungsgemäße Nanofaservlies stellt für in dieses eingebrachte Zellen eine dreidimensionale Umgebung zur Verfügung und die hohe Flexibilität der bereitgestellten Nanofasernstruktur erlaubt im weiteren Verlauf des Zellbesiedelungsprozesses ein Zusammenwachsen von Porenwänden. Die erfindungsgemäßen Nanofaservliese zeichnen sich daher durch die Fähigkeit aus, eine gute und schnelle Zellmigration und -besiedlung des gesamten Nanofaservlieses in kurzer Zeit, zum Beispiel innerhalb von 2 Wochen, zu ermöglichen. Das hochporöse erfindungsgemäße Nanofaservlies zeigt ähnliche Eigenschaften wie dezellularisiertes biologisches Gewebe: befindet sich erfindungsgemäß ausreichend Flüssigkeit, insbesondere Wasser, innerhalb des erfindungsgemäßen Nanofaservlies, insbesondere der Faserstruktur, ist dieses hoch flexibel und zeigt einen Gel-artigen Charakter. Reduziert sich der Wassergehalt, sind die mechanischen Eigenschaften eher vergleichbar mit Fasern. Durch die Kombination eines Elektrospinn-Prozesses mit weiteren additiven Fertigungsmethoden werden Strukturen im höheren Mikrometer-Maßstab aus (wasser-)löslichen Materialien durch ein weiteres Verfahren zwischen die Nanofasern als Porogen eingearbeitet. Dies ermöglicht die standardisierbare Herstellung von synthetischen Trägerstrukturen für 3D-Stromagewebe mit sehr hohen und kontrollierbaren Porositäten.

In einer besonders bevorzugten Ausführungsform liegt das erfindungsgemäße Nanofaservlies zusammen mit einer Flüssigkeit vor, insbesondere in feuchter und/oder flüssigkeitsgetränkter Form.

In besonders bevorzugter Ausführungsform liegt in dem erfindungsgemäßen Nanofaservlies mindestens eine Flüssigkeit vor.

In einer bevorzugten Ausführungsform ist die mindestens eine Flüssigkeit, welche in einem erfindungsgemäßen Nanofaservlies vorliegt, in dem Nanofaservlies sorbiert, insbesondere absorbiert und/oder adsorbiert, insbesondere absorbiert. In einer bevorzugten Ausführungsform ist die Flüssigkeit in dem erfindungsgemäßen Nanofaservlies in einem Volumen sorbiert, dass dieses Volumen dem Volumen der maximalen Flüssigkeitskapazität des Nanofaservlieses entspricht.

In besonders bevorzugter Ausführungsform beträgt in dem erfindungsgemäßen Nanofaservlies, vorzugsweise hergestellt durch ein erfindungsgemäßes erstes und/oder zweites Verfahren, das Volumen der mindestens einen Flüssigkeit 90,0 bis 99,9 Vol.-%, insbesondere 92,5 bis 99,9 Vol.-%, insbesondere 95,0 bis 99,9 Vol.-%, insbesondere 98,5 % bis 99,9 Vol.-% (jeweils bezogen auf Gesamtvolumen des mindestens eine Flüssigkeit aufweisenden Nanofaservlieses).

In besonders bevorzugter Ausführungsform beträgt in dem erfindungsgemäßen Nanofaservlies, vorzugsweise hergestellt durch ein erfindungsgemäßes erstes und/oder zweites Verfahren, das Gewicht der mindestens einen Flüssigkeit 90,0 bis 99,9 Gew.-%, insbesondere 92,5 bis 99,9 Gew.-%, insbesondere 95,0 bis 99,9 Gew.-%, insbesondere 98,5 % bis 99,9 Gew.-% (jeweils bezogen auf Gesamtgewicht des mindestens eine Flüssigkeit aufweisenden Nanofaservlieses).

In besonders bevorzugter Ausführungsform der vorliegenden Erfindung beträgt der Trockensubstanzgehalt des erfindungsgemäß bevorzugt mindestens eine Flüssigkeit aufweisenden Nanofaservlieses, vorzugsweise hergestellt durch ein erfindungsgemäßes erstes und/oder zweites Verfahren, 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils bezogen auf Gesamtgewicht des mindestens eine Flüssigkeit aufweisenden Nanofaservlieses).

In besonders bevorzugter Ausführungsform der vorliegenden Erfindung ist der Trockensubstanzgehalt des erfindungsgemäß mindestens eine Flüssigkeit aufweisenden Nanofaservlieses der Trockensubstanzgehalt der Nanofasern oder der Nanofasern und der gegebenenfalls vorhandenen Porogene, insbesondere der Nanofasern des Faservlieses.

Die Erfindung betrifft in bevorzugter Ausführungsform ein Nanofaservlies, vorzugsweise hergestellt durch ein erfindungsgemäßes erstes und/oder zweites Verfahren, wobei in dem Nanofaservlies mindestens eine Flüssigkeit, insbesondere mit einem Volumen von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% Flüssigkeit (jeweils bezogen auf das Gesamtvolumen des Nanofaservlieses), vorliegt und wobei der Trockensubstanzgehalt des erfindungsgemäß bevorzugt mindestens eine Flüssigkeit aufweisenden Nanofaservlieses 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils bezogen auf Gesamtgewicht des mindestens eine Flüssigkeit aufweisenden Nanofaservlieses) beträgt.

In besonders bevorzugter Ausführungsform beträgt das Gewicht der mindestens einen Flüssigkeit in dem erfindungsgemäßen Nanofaservlies, vorzugsweise hergestellt durch ein erfindungsgemäßes erstes und/oder zweites Verfahren, 90,0 bis 99,9 Gew.-%, insbesondere 92,5 bis 99,9 Gew.-%, insbesondere 95,0 bis 99,9 Gew.-%, insbesondere 98,5 % bis 99,9 Gew.-% (jeweils bezogen auf Gesamtgewicht des mindestens eine Flüssigkeit aufweisenden Nanofaservlieses) und das Gewicht der Nanofasern des Nanofaservlieses 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils Trockensubstanzgehalt der Nanofaser, jeweils bezogen auf Gesamtgewicht des mindestens eine Flüssigkeit aufweisenden Nanofaservlieses), wobei bevorzugt das Gewicht der mindestens einen Flüssigkeit mit dem Gewicht der Nanofasern auf 100 Gewichtsprozent aufaddiert.

Die Erfindung betrifft in bevorzugter Ausführungsform ein Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses), wobei in dem Nanofaservlies mindestens eine Flüssigkeit, insbesondere mit einem Volumen von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% Flüssigkeit (jeweils bezogen auf das Gesamtvolumen des Nanofaservlieses) vorliegt.

In besonders bevorzugter Ausführungsform beträgt in dem erfindungsgemäßen Nanofaservlies mit einer Porosität von 90,0 bis 99,9 Vol.-% das Gewicht der mindestens einen Flüssigkeit 90,0 bis 99,9 Gew.-%, insbesondere 92,5 bis 99,9 Gew.-%, insbesondere 95,0 bis 99,9 Gew.-%, insbesondere 98,5 % bis 99,9 Gew.-% (jeweils bezogen auf Gesamtgewicht des mindestens eine Flüssigkeit aufweisenden Nanofaservlieses).

In besonders bevorzugter Ausführungsform der vorliegenden Erfindung beträgt der Trockensubstanzgehalt des erfindungsgemäß bevorzugt mindestens eine Flüssigkeit aufweisenden Nanofaservlieses mit einer Porosität von 90,0 bis 99,9 Vol.-% 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils bezogen auf Gesamtgewicht des mindestens eine Flüssigkeit aufweisenden Nanofaservlieses).

Die Erfindung betrifft in bevorzugter Ausführungsform ein Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses), wobei in dem Nanofaservlies mindestens eine Flüssigkeit, insbesondere mit einem Volumen von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% Flüssigkeit (jeweils bezogen auf das Gesamtvolumen des Nanofaservlieses), vorliegt und wobei der Trockensubstanzgehalt des erfindungsgemäß bevorzugt mindestens eine Flüssigkeit aufweisenden Nanofaservlieses 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils bezogen auf Gesamtgewicht des mindestens eine Flüssigkeit aufweisenden Nanofaservlieses) beträgt.

In besonders bevorzugter Ausführungsform beträgt das Gewicht der mindestens einen Flüssigkeit in dem erfindungsgemäßen Nanofaservlies mit einer Porosität von 90,0 bis 99,9 Vol.-% 90,0 bis 99,9 Gew.-%, insbesondere 92,5 bis 99,9 Gew.-%, insbesondere 95,0 bis 99,9 Gew.-%, insbesondere 98,5 % bis 99,9 Gew.-% (jeweils bezogen auf Gesamtgewicht des mindestens eine Flüssigkeit aufweisenden Nanofaservlieses) und das Gewicht der Nanofasern des Nanofaservlieses 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils Trockensubstanzgehalt der Nanofaser, jeweils bezogen auf Gesamtgewicht des mindestens eine Flüssigkeit aufweisenden Nanofaservlieses), wobei bevorzugt das Gewicht der mindestens einen Flüssigkeit mit dem Gewicht der Nanofasern auf 100 Gewichtsprozent aufaddiert.

In besonders bevorzugter Ausführungsform ist die Flüssigkeit, welche in einem erfindungsgemäßen Nanofaservlies vorliegt, eine Flüssigkeit, die die aus dem Nanofasermaterial gebildeten Nanofasern nicht angreift, insbesondere nicht abbaut, nicht strukturell ändert oder zerstört.

In besonders bevorzugter Ausführungsform ist die Flüssigkeit, welche in einem erfindungsgemäßen Nanofaservlies vorliegt, Wasser.

In besonders bevorzugter Ausführungsform ist die Flüssigkeit, welche in einem erfindungsgemäßen Nanofaservlies vorliegt, eine wässrige Lösung, insbesondere einer oder mehrerer Substanzen.

In einer bevorzugten Ausführungsform beträgt der pH-Wert der wässrigen Lösung, welche in einem erfindungsgemäßen Nanofaservlies vorliegt, 1 bis 13, insbesondere 1 bis 11, insbesondere 2 bis 11, insbesondere 3 bis 11, insbesondere 5 bis 9, vorzugsweise 6 bis 8, insbesondere 7.

In einer besonders bevorzugten Ausführungsform, wenn zur Herstellung des Nanofaservlieses Calciumcarbonat als Porogenmaterial verwendet wurde, das Porogen im Nanofaservlies vorliegt und das Nanofaservlies in einer wässrigen Lösung vorliegt, beträgt der pH-Wert dieser wässrigen Lösung 4 bis 5, insbesondere 4,5.

In einer besonders bevorzugten Ausführungsform, wenn zur Herstellung des Nanofaservlieses Chitosan als Porogenmaterial verwendet wurde, das Porogen im Nanofaservlies vorliegt und das Nanofaservlies in einer wässrigen Lösung vorliegt, beträgt der pH-Wert dieser wässrigen Lösung 2 bis 4, insbesondere 3.

In einer besonders bevorzugten Ausführungsform, wenn zur Herstellung des Nanofaservlieses Zinkoxid als Porogenmaterial verwendet wurde, das Porogen im Nanofaservlies vorliegt und das Nanofaservlies in einer wässrigen Lösung vorliegt, beträgt der pH-Wert dieser wässrigen Lösung 1 bis 3, insbesondere 2.

In einer besonders bevorzugten Ausführungsform, wenn zur Herstellung des Nanofaservlieses Kieselgel/amorphes Silica als Porogenmaterial verwendet wurde, das Porogen im Nanofaservlies vorliegt und das Nanofaservlies in einer wässrigen Lösung vorliegt, beträgt der pH-Wert dieser wässrigen Lösung 11 bis 13, insbesondere 12.

In einer bevorzugten Ausführungsform ist die Flüssigkeit, welche in einem erfindungsgemäßen Nanofaservlies vorliegt, eine gepufferte Lösung, insbesondere PBS (Phosphate buffered Saline), insbesondere PBS+ (PBS mit $Ca^{2+}$- und $Mg^{2+}$-Ionen). Insbesondere weist die gepufferte Lösung als Puffer-System Bicarbonat-Puffer, Citrat-Puffer und/oder Acetat-Puffer auf.

In besonders bevorzugter Ausführungsform ist die Flüssigkeit, welche in einem erfindungsgemäßen Nanofaservlies vorliegt, eine nicht-wässrige Lösung, insbesondere einer oder mehrerer Substanzen.

In einer bevorzugten Ausführungsform ist die Flüssigkeit, welche in einem erfindungsgemäßen Nanofaservlies vorliegt, Ethanol.

In einer bevorzugten Ausführungsform ist die Flüssigkeit, welche in einem erfindungsgemäßen Nanofaservlies vorliegt, Isopropanol.

In einer bevorzugten Ausführungsform ist die Flüssigkeit, welche in einem erfindungsgemäßen Nanofaservlies vorliegt, Dimethylsulfoxid.

In besonders bevorzugter Ausführungsform ist die Flüssigkeit, welche in einem erfindungsgemäßen Nanofaservlies vorliegt, eine nicht-wässrige oder wässrige Lösung einer gegenüber dem Nanofasermaterial inerten Substanz oder Substanzgemisch in einem Lösungsmittel, zum Beispiel Wasser.

In einer besonders bevorzugten Ausführungsform sind die Poren in einem erfindungsgemäßen Nanofaservlies homogen oder hierarchisch strukturiert verteilt.

In einer besonders bevorzugten Ausführungsform umfasst das Nanofaservlies, vorzugsweise hergestellt durch ein erfindungsgemäßes erstes und/oder zweites Verfahren, zusätzlich Porogene.

In einer besonders bevorzugten Ausführungsform sind die Porogene partikelförmig oder faserförmig.

In einer besonders bevorzugten Ausführungsform weisen die Porogene einen Durchmesser von 30 bis 1000 μm, insbesondere 40 bis 1000 μm, insbesondere 50 bis 1000 μm, insbesondere 70 bis 1000 μm, insbesondere 100 bis 1000 μm, insbesondere 30 bis 500 μm, insbesondere 40 bis 500 μm, insbesondere 50 bis 500 μm, insbesondere 70 bis 500 μm, insbesondere 100 bis 500 μm, insbesondere 30 bis 250 μm, insbesondere 40 bis 250 μm, insbesondere 50 bis 250 μm, insbesondere 70 bis 250 μm, insbesondere 100 bis 250 μm, insbesondere 30 bis 150 μm, insbesondere 40 bis 150 μm, insbesondere 50 bis 150 μm, insbesondere 70 bis 150 μm, insbesondere 100 bis 150 μm, auf.

In einer besonders bevorzugten Ausführungsform weisen die Porogene einen Durchmesser von 30 bis 1000 μm, insbesondere 40 bis 1000 μm, insbesondere 50 bis 1000 μm, insbesondere 70 bis 1000 μm, insbesondere 100 bis 1000 μm, insbesondere 30 bis 500 μm, insbesondere 40 bis 500 μm, insbesondere 50 bis 500 μm, insbesondere 70 bis 500 μm, insbesondere 100 bis 500 μm, insbesondere 30 bis 250 μm, insbesondere 40 bis 250 μm, insbesondere 50 bis 250 μm, insbesondere 70 bis 250 μm, insbesondere 100 bis 250 μm, insbesondere 30 bis 150 μm, insbesondere 40 bis 150 μm, insbesondere 50 bis 150 μm, insbesondere 70 bis 150 μm, insbesondere 100 bis 150 μm, auf, wobei mindestens 70 %, insbesondere mindestens 80 %, insbesondere mindestens 90 %, insbesondere mindestens 95 %, insbesondere mindestens 99 %, insbesondere 100 %, der Porogene einen Durchmesser von mindestens 30 μm, insbesondere mindestens 40 μm, insbesondere mindestens 50 μm, insbesondere mindestens 70 μm, insbesondere mindestens 100 μm, aufweisen.

In einer besonders bevorzugten Ausführungsform weisen mindestens 70 %, insbesondere mindestens 80 %, insbesondere mindestens 90 %, insbesondere mindestens 95 %, insbesondere mindestens 99 %, insbesondere 100 %, der Porogene einen Durchmesser von 30 bis 1000 μm, insbesondere 40 bis 1000 μm, insbesondere 50 bis 1000 μm, insbesondere 70 bis 1000 μm, insbesondere 100 bis 1000 μm, insbesondere 30 bis 500 μm, insbesondere 40 bis 500 μm, insbesondere 50 bis 500 μm, insbesondere 70 bis 500 μm, insbesondere 100 bis 500 μm, insbesondere 30 bis 250 μm, insbesondere 40 bis 250 μm, insbesondere 50 bis 250 μm, insbesondere 70 bis 250 μm, insbesondere 100 bis 250 μm, insbesondere 30 bis 150 μm, insbesondere 40 bis 150 μm, insbesondere 50 bis 150 μm, insbesondere 70 bis 150 μm, insbesondere 100 bis 150 μm, auf.

In einer besonders bevorzugten Ausführungsform der vorliegenden Erfindung liegen in dem erfindungsgemäßen Nanofaservlies mindestens eine Flüssigkeit und Porogene vor.

In einer besonders bevorzugten Ausführungsform der vorliegenden Erfindung liegen in dem erfindungsgemäßen Nanofaservlies, insbesondere hergestellt durch ein erfindungsgemäßes erstes und/oder zweites Verfahren, Porogene vor, wobei das Gesamtgewicht der Porogene 0,07 bis 99,997 Gew.-%, insbesondere 10,0 bis 99,99 Gew.-%, insbesondere 50,0 bis 99,95 Gew.-%, insbesondere 80,0 bis 99,95 Gew.-% (jeweils bezogen auf Gesamtgewicht des Nanofaservlieses) beträgt.

Die Erfindung betrifft in bevorzugter Ausführungsform ein Nanofaservlies, vorzugsweise hergestellt durch ein erfindungsgemäßes erstes und/oder zweites Verfahren, wobei in dem Nanofaservlies Porogene, insbesondere mit einem Volumen von 40,0 bis 99,9 Vol.-%, insbesondere 50,0 bis 99,9 Vol.-%, insbesondere 60,0 bis 99,9 Vol.-%, insbesondere 70,0 bis 99,9 Vol.-%, insbesondere 80,0 bis 99,9 Vol.-%, insbesondere 90,0 bis 99,9 Vol.-%, insbesondere 40,0 bis 95,0 Vol.-%, insbesondere 50,0 bis 95,0 Vol.-%, insbesondere 60,0 bis 95,0 Vol.-%, insbesondere 70,0 bis 95,0 Vol.-%,

insbesondere 80,0 bis 95,0 Vol.-%, insbesondere 90,0 bis 95,0 Vol.-%, insbesondere 92,5 bis 95,0 Vol.-%, Porogene (jeweils bezogen auf das Gesamtvolumen des Nanofaservlieses) vorliegen.

In einer besonders bevorzugten Ausführungsform der vorliegenden Erfindung liegen in dem erfindungsgemäßen Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses), Porogene vor, wobei das Gesamtvolumen der Porogene 40,0 bis 95,0 Vol.-%, insbesondere 50,0 bis 95,0 Vol.-%, insbesondere 60,0 bis 95,0 Vol.-%, insbesondere 70,0 % bis 95,0 Vol.-%, insbesondere 80,0 bis 95,0 Vol.-%, insbesondere 90,0 bis 95,0 Vol.-%, insbesondere 40,0 bis 90,0 Vol.-%, insbesondere 50,0 bis 90,0 Vol.-%, insbesondere 60,0 bis 90,0 Vol.-%, insbesondere 70,0 bis 90,0 Vol.-%, insbesondere 80,0 bis 90,0 Vol.-% (jeweils bezogen auf Gesamtvolumen des Nanofaservlieses) beträgt.

In einer besonders bevorzugten Ausführungsform der vorliegenden Erfindung liegen in dem erfindungsgemäßen Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses), Porogene vor, wobei das Gesamtgewicht der Porogene 0,07 bis 99,997 Gew.-%, insbesondere 10,0 bis 99,99 Gew.-%, insbesondere 50,0 bis 99,95 Gew.-%, insbesondere 80,0 bis 99,95 Gew.-% (jeweils bezogen auf Gesamtgewicht des Nanofaservlieses) beträgt.

Die Erfindung betrifft in bevorzugter Ausführungsform ein Nanofaservlies, wobei in dem Nanofaservlies mindestens eine Flüssigkeit und Porogene vorliegen, insbesondere mit einem Volumen von 40,0 bis 99,9 Vol.-%, insbesondere 50,0 bis 99,9 Vol.-%, insbesondere 60,0 bis 99,9 Vol.-%, insbesondere 70,0 bis 99,9 Vol.-%, insbesondere 80,0 bis 99,9 Vol.-%, insbesondere 90,0 bis 99,9 Vol.-%, insbesondere 40,0 bis 95,0 Vol.-%, insbesondere 50,0 bis 95,0 Vol.-%, insbesondere 60,0 bis 95,0 Vol.-%, insbesondere 70,0 bis 95,0 Vol.-%, insbesondere 80,0 bis 95,0 Vol.-%, insbesondere 90,0 bis 95,0 Vol.-%, insbesondere 92,5 bis 95,0 Vol.-%, Porogene (jeweils bezogen auf das Gesamtvolumen des Nanofaservlieses).

In einer besonders bevorzugten Ausführungsform der vorliegenden Erfindung liegen in dem erfindungsgemäßen Nanofaservlies, vorzugsweise hergestellt durch ein erfindungsgemäßes erstes und/oder zweites Verfahren, Porogene und mindestens eine Flüssigkeit vor, wobei das Gesamtvolumen der mindestens einen Flüssigkeit und der Porogene 90,0 bis 99,9 Vol.-%, insbesondere 92,5 bis 99,9 Vol.-%, insbesondere 95,0 bis 99,9 Vol.-%, insbesondere 98,5 % bis 99,9 Vol.-% (jeweils bezogen auf Gesamtvolumen des Nanofaservlieses) beträgt.

In einer besonders bevorzugten Ausführungsform der vorliegenden Erfindung liegen in dem erfindungsgemäßen Nanofaservlies, vorzugsweise hergestellt durch ein erfindungsgemäßes erstes und/oder zweites Verfahren, Porogene und mindestens eine Flüssigkeit vor, wobei das Gesamtgewicht der mindestens einen Flüssigkeit und der Porogene 90,00 bis 99,99 Gew.-%, insbesondere 92,50 bis 99,99 Gew.-%, insbesondere 95,00 bis 99,99 Gew.-%, insbesondere 98,50 % bis 99,99 Gew.-% (jeweils bezogen auf Gesamtgewicht des Nanofaservlieses) beträgt.

Die Erfindung betrifft in bevorzugter Ausführungsform ein Nanofaservlies, vorzugsweise hergestellt durch ein erfindungsgemäßes erstes und/oder zweites Verfahren, wobei in dem Nanofaservlies mindestens eine Flüssigkeit und Porogene insbesondere mit einem Volumen von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% an Porogenen und mindestens einer Flüssigkeit (jeweils bezogen auf das Gesamtvolumen des Nanofaservlieses) vorliegen und wobei der Trockensubstanzgehalt des Nanofaservlieses 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils bezogen auf Gesamtgewicht des mindestens eine Flüssigkeit aufweisenden Nanofaservlieses) beträgt.

In einer besonders bevorzugten Ausführungsform der vorliegenden Erfindung liegen in dem erfindungsgemäßen Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses) Porogene und mindestens eine Flüssigkeit vor, wobei das Gesamtvolumen der Flüssigkeit und Porogene 90,0 bis 99,9 Vol.-%, insbesondere 92,5 bis 99,9 Vol.-%, insbesondere 95,0 bis 99,9 Vol.-%, insbesondere 98,5 % bis 99,9 Vol.-% (jeweils bezogen auf Gesamtvolumen des Nanofaservlieses) beträgt.

In einer besonders bevorzugten Ausführungsform der vorliegenden Erfindung liegen in dem erfindungsgemäßen Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses) Porogene und mindestens eine Flüssigkeit vor, wobei das Gesamtgewicht der mindestens einen Flüssigkeit und Porogene 90,00 bis 99,99 Gew.-%, insbesondere 92,50 bis 99,99 Gew.-%, insbesondere 95,00 bis 99,99 Gew.-%, insbesondere 98,50 % bis 99,99 Gew.-% (jeweils bezogen auf Gesamtgewicht des Nanofaservlieses) beträgt.

Die Erfindung betrifft in bevorzugter Ausführungsform ein Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses), wobei in dem Nanofaservlies mindestens eine Flüssigkeit und Porogene, insbesondere mit einem Volumen von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% an Porogenen und Flüssigkeit (jeweils bezogen auf das Gesamtvolumen des Nanofaservlieses) vorliegen und wobei der Trockensubstanzgehalt des Nanofaservlieses 0,1 bis 10,0 Gew.-%, insbesondere 0,1 bis 7,5 Gew.-%, insbesondere 0,1 bis 5,0 Gew.-%, insbesondere 0,1 bis 1,5 Gew.-% (jeweils bezogen auf Gesamtgewicht des mindestens eine Flüssigkeit aufweisenden Nanofaservlieses) beträgt.

In einer besonders bevorzugten Ausführungsform umfasst das Nanofaservlies, vorzugsweise hergestellt durch ein erfindungsgemäßes erstes und/oder zweites Verfahren, keine Porogene.

In einer besonders bevorzugten Ausführungsform der vorliegenden Erfindung liegen in dem Nanofaservlies mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses) keine Porogene vor.

Bevorzugt weisen die Nanofasern einen Durchmesser von 100 bis 1000 nm, insbesondere 200 bis 1000 nm, insbesondere 300 bis 1000 nm, insbesondere 100 bis 900 nm, insbesondere 200 bis 900 nm, insbesondere 300 bis 900 nm, insbesondere 100 bis 800 nm, insbesondere 200 bis 800 nm, insbesondere 300 bis 800 nm, insbesondere 100 bis 700 nm, insbesondere 200 bis 700 nm, insbesondere 300 bis 700 nm, auf.

Die Nanofasern des Nanofaservlieses liegen in einem erfindungsgemäßen Nanofaservlies in Form von Nanofaserlagen vor.

In besonders bevorzugter Ausführungsform ist das Netzwerk der Nanofasern ein flexibles Netzwerk, insbesondere aufgebaut aus Nanofaserlagen.

Im Zusammenhang mit der vorliegenden Erfindung wird unter einer "Faserlage" oder "Nanofaserlage" eine Schicht aus über- und/oder nebeneinander, insbesondere übereinander angeordneten Nanofasern verstanden, insbesondere sind einzelne Nanofaserlagen voneinander durch die Poren getrennt. Bevorzugt werden die Nanofasern und Nanofaserlagen des erfindungsgemäßen Nanofaservlieses durch ein erfindungsgemäßes Verfahren zur Herstellung eines Nanofaservlieses, insbesondere dessen Verfahrensschritt b) oder b'), erhalten.

In einer bevorzugten Ausführungsform umfassen, insbesondere bestehen, die Faserlagen aus 1 bis 25 Fasern, insbesondere 3 bis 20 Fasern, insbesondere 3 bis 15 Fasern, insbesondere 3 bis 10 Fasern, welche über- und/oder nebeneinander, insbesondere übereinander, angeordnet sind.

In einer besonders bevorzugten Ausführungsform umfassen, insbesondere bestehen, die Faserlagen aus 1 bis 25 Fasern, insbesondere 3 bis 20 Fasern, insbesondere 3 bis 15 Fasern, insbesondere 3 bis 10 Fasern, welche über- und/oder nebeneinander, insbesondere übereinander, angeordnet sind, jeweils bezogen auf eine Tiefe des Nanofaservlies von 10 $\mu$m.

In einer bevorzugten Ausführungsform haben die Fasern in der Faserlage keinen dauerhaften Kontakt und sind in alle Raumrichtungen frei beweglich.

Der Aufbau eines erfindungsgemäßen Nanofaservlieses umfasst bevorzugt mindestens zwei, bevorzugt mehrere einzelne Nanofaserlagen, welche bevorzugt durch die Poren, bevorzugt - erzeugt durch die erfindungsgemäß bevorzugt vorgesehene Entfernung von Porogenen, getrennt werden. Je nach erfindungsgemäß bevorzugter Herstellung können diese Poren durch die Nanofaserlagen abgeschlossen sein oder sehr lang bis unendlich einen laminaren Charakter aufweisen.

Innerhalb der einzelnen Nanofaserlagen werden erfindungsgemäß die Freiräume, senkrecht zur Hauptausdehnungsfläche des Nanofaservlieses, zwischen den Nanofasern als Maschen bezeichnet.

Im Zusammenhang mit der vorliegenden Erfindung wird unter einer "Masche" der Zwischenraum zwischen den einzelnen Nanofasern eines erfindungsgemäßen Nanofaservlieses verstanden, insbesondere der Zwischenraum, der nicht durch ein erfindungsgemäß während oder nach der Herstellung des Nanofaservlieses vorgesehenes Entfernen eines Porogens entstanden ist. Insbesondere werden unter Maschen die Freiräume verstanden, welche sich senkrecht zur Hauptausdehnungsfläche des Nanofaservlieses befinden.

Eine Masche bildet sich durch das Über- und/oder Nebeneinanderlegen von einzelnen Nanofasern zu Nanofaserlagen. Die entstehende Masche wird über die gebildete Durchtrittsfläche über die Dicke der einzelnen Nanofaserlagen bestimmt.

Die Maschen sind insofern von Bedeutung, als dass Zellen diese Maschen durchwandern können. Daher wird die Größe der "Masche" erfindungsgemäß auch als Fläche (Durchtrittsfläche mit definierter Maschenweite) angegeben.

Die Maschenweite hängt unter anderem ab von der Anzahl an Nanofaserlagen übereinander. Je mehr Nanofaserlagen übereinanderliegen, desto kleiner werden die Maschen, also die Maschenweite, und desto fester sind die Nanofasern untereinander verklemmt, was beides die Zugänglichkeit für Zellen reduziert.

In einer bevorzugten Ausführungsform weist das Nanofaservlies Maschen mit einer Maschenweiten von maximal 200 $\mu$m$^2$, insbesondere maximal 150 $\mu$m$^2$, insbesondere maximal 100 $\mu$m$^2$, insbesondere maximal 50 $\mu$m$^2$ auf.

In bevorzugter Ausführungsform beträgt die Maschenweite 10 bis 200 $\mu$m$^2$, insbesondere 20 bis 200 $\mu$m$^2$, insbesondere 50 bis 200 $\mu$m$^2$, insbesondere 100 bis 200 $\mu$m$^2$, insbesondere 150 bis 200 $\mu$m$^2$, insbesondere 10 bis 150 $\mu$m$^2$, insbesondere 20 bis 150 $\mu$m$^2$, insbesondere 50 bis 150 $\mu$m$^2$, insbesondere 100 bis 150 $\mu$m$^2$, insbesondere 10 bis 100 $\mu$m$^2$, insbesondere 20 bis 100 $\mu$m$^2$, insbesondere 50 bis 100 $\mu$m$^2$, insbesondere 10 bis 50 $\mu$m$^2$, insbesondere 20 bis 50 $\mu$m$^2$. Insbesondere weist das Nanofaservlies Maschen mit einer Maschenweite entsprechend dieses Absatzes auf.

In einer bevorzugten Ausführungsform weist das Nanofaservlies 3000 bis 8000 Maschen pro mm$^2$, insbesondere 3500 bis 7000 Maschen pro mm$^2$, insbesondere 3000 bis 6000 Maschen pro mm$^2$, insbesondere 3100 bis 5000 Maschen pro mm², insbesondere 3200 bis 4800 Maschen pro mm², insbesondere 3500 bis 4500 Maschen pro mm², auf. In einer besonders bevorzugten Ausführungsform werden bei der Bestimmung der Maschen pro mm² nur Maschen mit einer minimalen Maschenweite von 0,5 μm² bestimmt.

Im Zusammenhang mit der vorliegenden Erfindung wird unter einer "Pore" ein von Nanofaserlagen umschlossener Raum verstanden, welcher durch Porogene, Zellen oder Fluide, also Flüssigkeiten oder Gase, füllbar ist. Die Poren trennen die einzelnen Nanofaserlagen voneinander räumlich.

Eine Pore erstreckt sich innerhalb des erfindungsgemäßen Nanofaservlies in x-y-z-Richtung (Breite, Tiefe, Höhe der Pore), wobei Tiefe sowie Breite der Poren Flächenausdehnungsrichtungen des Nanofaservlieses beschreiben. Die Höhe der Poren erstreckt sich senkrecht zu den Flächenausdehnungsrichtungen des Nanofaservlieses und ist bevorzugt in Richtung der Höhe des Nanofaservlieses (erfindungsgemäß auch als Dicke des Nanofaservlies bezeichnet) angeordnet. Die Breite beziehungsweise Tiefe der Poren sind definiert als Richtungen parallel zur Ausdehnungsrichtungen des Nanofaservlieses.

Der Zweck der Poren ist auch, die einzelnen Nanofaserlagen voneinander zu trennen und damit die bevorzugte hohe Maschenweite zu stabilisieren. Fällt die Porenstruktur zusammen, verringert sich auch die Maschenweite und zerstört damit die Dreidimensionalität für die Zellbesiedelung. Die Breite, Tiefe und Höhe der Poren wird insbesondere ohne mechanische Belastung des Nanofaservlieses bestimmt, insbesondere ohne Einspannung des Nanofaservlieses in eine Vorrichtung, bevorzugt direkt nach dem Entfernen des Porogens.

Die Höhe der Poren entspricht bevorzugt dem Durchmesser des Porogens und kann sich gegebenenfalls durch die Bewegung des, insbesondere flexiblen, erfindungsgemäßen Nanofaservlieses verändern (Stauchung des Nanofaservlieses führt zu Vergrößerung der Porenhöhe; Zugspannung am Nanofaservlies führt zu Verkleinerung der Porenhöhe).

Die Breite beziehungsweise Tiefe der Poren ist bevorzugt um ein Vielfaches größer als der Durchmesser des Porogens, da die Nanofasern im Spinnprozess sich in gespanntem Zustand über das Porogen ablegen. Je höher dabei die Rotationsgeschwindigkeit des Targets ist (Faserorientierung), desto weiter werden die Nanofasern aufgespannt und desto breiter wird die Pore. Im Extremfall befindet sich das nächste Porogen nah genug, dass die Nanofasern die untere Nanofaserlage nicht mehr berühren und über den Porogenen aufgespannt werden. Dies führt zu einem lamellaren Aufbau, wodurch die Poren in mindestens einer Ausdehnungsrichtung (zum Beispiel Breite) unendlich groß werden.

Bevorzugt ist diese unendliche Porenausdehnung nur in einer Richtung vorliegend. Die andere Ausdehnungsrichtung (zum Beispiel Tiefe) bleibt bevorzugt begrenzt. Diese Begrenzung ist abhängig von der Porogengröße.

Bevorzugt ist eine maximale Porenbreite, die dem 3 bis 5-fachen Porogendurchmesser entspricht.

Bevorzugt ist eine maximale Porentiefe, die dem 3 bis 5-fachen Porogendurchmesser entspricht. Sehr große Porogene führen bevorzugt zu einem Schichtaufbau, da hier die Nanofasern in jeder Ausdehnungsrichtung über die Porogene gespannt werden. Dieser Schichtaufbau führt bevorzugt zu einer effektiven Trennung der einzelnen Nanofaserlagen und beeinträchtigt nicht die Besiedelung mit Zellen.

Bevorzugte Ausführungsformen stellen Poren dar mit einer Breite und/oder Tiefe von 150 bis 250 μm, insbesondere, wenn die für die Herstellung der erfindungsgemäßen Nanofaservliese eingesetzten Porogene 20 bis 100 μm, insbesondere 50 μm, Durchmesser aufweisen.

Bevorzugte Ausführungsformen sind Poren mit einer Breite und/oder Tiefe von 1500 bis 2500 μm, insbesondere, wenn die für die Herstellung der erfindungsgemäßen Nanofaservliese eingesetzten Porogene 200 bis 1000 μm, insbesondere 500 μm, Durchmesser aufweisen.

In bevorzugter Ausführungsform beträgt die Porentiefe 50 bis 5000 μm, insbesondere 100 bis 5000 μm, insbesondere 50 bis 2000 μm, insbesondere 100 bis 2000 μm, insbesondere 50 bis 1000 μm, insbesondere 100 bis 1000 μm, insbesondere 50 bis 500 μm, insbesondere 100 bis 500 μm.

In bevorzugter Ausführungsform beträgt die Porenbreite 50 bis 5000 μm, insbesondere 100 bis 5000 μm, insbesondere 50 bis 2000 μm, insbesondere 100 bis 2000 μm, insbesondere 50 bis 1000 μm, insbesondere 100 bis 1000 μm, insbesondere 50 bis 500 μm, insbesondere 100 bis 500 μm.

In bevorzugter Ausführungsform beträgt die Porenhöhe 20 bis 1000 μm, insbesondere 50 bis 1000 μm, insbesondere 20 bis 500 μm, insbesondere 50 bis 500 μm.

Im Zusammenhang mit der vorliegenden Erfindung wird unter "Porosität" der prozentuale Anteil am Gesamtvolumen des Nanofaservlieses verstanden, welcher durch das Volumen der Poren und der Maschen gebildet wird. Die Volumina der Poren können mit Flüssigkeit und/oder Porogenen gefüllt sein.

In bevorzugter Weise liegt die erfindungsgemäß vorgesehene Porosität des Nanofaservlieses vor, wenn das Nanofaservlies in Flüssigkeit, insbesondere mit einem Volumen von 90,0 bis 99,9 Vol.-%, insbesondere 92,5 bis 99,9 Vol.-%, insbesondere 98,5 bis 99,9 Vol.-% an Flüssigkeit (jeweils bezogen auf das Gesamtvolumen des Nanofaservlieses), vorliegt, insbesondere ohne mechanische Belastung, insbesondere Zug- oder Druckbelastung vorliegt.

In bevorzugter Weise liegt die erfindungsgemäß vorgesehene Porosität des Nanofaservlieses vor, wenn das Nanofaservlies in Flüssigkeit und mit Porogenen, insbesondere mit einem Volumen von 90,0 bis 99,9 Vol.-%, insbesondere 92,5 bis 99,9 Vol.-%, insbesondere 98,5 bis 99,9 Vol.-% an Porogenen und Flüssigkeit (jeweils bezogen auf das Gesamtvolumen des Nanofaservlieses), vorliegt, insbesondere ohne mechanische Belastung, insbesondere Zug- oder Druckbelastung vorliegt.

Insbesondere wird die Porosität in Vol.-% bestimmt gemäß Formel (A), insbesondere, ohne dass das Nanofaservlies einer mechanischen Belastung, insbesondere Zug- oder Druckbelastung, während der Porositätsermittlung ausgesetzt wird.

$$\text{Formel (A):}$$

$$\text{Porosität P} = (V_{Gesamt} - V_{Fasern}) / V_{Gesamt} \times 100,$$

wobei $V_{Gesamt}$ das Volumen des gesamten Nanofaservlieses und $V_{Fasern}$ das Volumen der gesamten in dem Nanofaservlies vorhandenen Fasern darstellt.

Insbesondere wird die Porosität in Vol.-% bestimmt gemäß Formel (B), insbesondere, ohne dass das Nanofaservlies einer mechanischen Belastung, insbesondere Zug- oder Druckbelastung während der Porositätsermittlung ausgesetzt wird.

$$\text{Porosität P} = (\text{Dicke}_{poröses} \text{ Faservlies} \times A_{makroskopisch} - m_{Faser}/\text{Dichte}_{Fasermaterial})/( \text{Dicke}_{poröses} \text{ Faservlies} \times A_{makroskopisch}) \times 100 \qquad \text{Formel (B):}$$

Besonders bevorzugt wird gemäß Formel (B) die Porosität eines Nanofaservlies daher durch die Ermittlung der Dicke (erfindungsgemäß auch als Höhe des Nanofaservlieses bezeichnet) eines Stücks des porösen Nanofaservlieses sowie der Messung der Trockenmasse (m), der Fasern des Stücks des Nanofaservlieses bestimmt. Anschließend kann über Einbeziehen der makroskopisch ermittelten Oberfläche (A) des Nanofaservlies-Stücks und der theoretischen Dichte des Nanofasermaterials, insbesondere Polymers, die Porosität in Vol.-%, bezogen auf das Gesamtvolumen des Nanofaservlieses, berechnet werden.

Im Zusammenhang mit der vorliegenden Erfindung wird unter der makroskopisch ermittelten "Oberfläche (A)" des Nanofaservlies-Stücks insbesondere die Oberfläche verstanden, welche durch die Breite und Tiefe (also die Flächenausdehnungsrichtungen) des Nanofaservlieses, insbesondere eines Stückes des Nanofaservlieses, gebildet wird.

Insbesondere wird die Porosität in Vol.-% bestimmt gemäß der Bestimmungsmethode nach Beispiel 2a, insbesondere, ohne dass das Nanofaservlies einer mechanischen Belastung, insbesondere Zug- oder Druckbelastung, während der Porositätsermittlung ausgesetzt wird.

Insbesondere wird die Porosität eines Nanofaservlieses erfindungsgemäß mittels konfokaler Reflexionsmikroskopie in einer wässrigen Lösung, insbesondere Wasser, ohne Porogen bestimmt, insbesondere wobei das Nanofaservlies ohne mechanische Belastung, insbesondere Zug- oder Druckbelastung vorliegt, abgesehen von dem hydrostatischen Druck der wässrigen Lösung und dem Atmosphärendruck. Bevorzugt wird die Porosität bestimmt gemäß der Bestimmungsmethode nach Beispiel 2b, insbesondere ohne dass das Nanofaservlies einer mechanischen Belastung, insbesondere Zug- oder Druckbelastung, während der Porositätsermittlung ausgesetzt wird.

In einer bevorzugten Ausführungsform der Bestimmung der Porosität eines Nanofaservlieses mittels konfokaler Reflexionsmikroskopie werden gegebenenfalls vorhandene Porogene aus dem Nanofaservliesstück entfernt und das Nanofaservlies wird mit der wässrigen Lösung, insbesondere Wasser, vollständig bedeckt. Anschließend werden optische Schnittbilder eines definierten Volumens des Nanofaservlieses mit dem konfokalen Reflexionsmikroskop erstellt. Anhand dieser optischen Schnittbilder wird das Volumen der Nanofasern ($V_{Fasern}$), insbesondere also das von den Nanofasern in den optischen Schnittbildern eingenommene Volumen, und das Volumen aller Freiräume zwischen den Nanofasern ($V_{Freiraum}$) bestimmt. Die Porosität kann dann mittels folgender Formel (C) berechnet werden:

$$\text{Formel (C):}$$

$$\text{Porosität P} = V_{Freiraum}/(V_{Freiraum} + V_{Fasern}) \times 100.$$

Insbesondere erfolgt die Bestimmung der Porosität mit einem konfokalen Mikroskop, insbesondere mittels konfokaler Reflexionsmikroskopie, bei einer Temperatur von 20 bis 25 °C, insbesondere 20 °C. Insbesondere wird die wässrige Lösung, in welcher das Nanofaservlies vorliegt, während der Bestimmung der Porosität temperiert, insbesondere auf 20 bis 25 °C, insbesondere 20 °C.

In besonders bevorzugter Ausführungsform der vorliegenden Erfindung wird die Porosität des erfindungsgemäßen Nanofaservlieses unmittelbar nach Entfernung des für dessen erfindungsgemäße Herstellung verwendeten Porogens, insbesondere sämtlicher Porogene, bestimmt, insbesondere innerhalb von 3 Tagen, insbesondere innerhalb von 1 Tag, insbesondere innerhalb von 8 Stunden, insbesondere innerhalb von 7 Stunden, insbesondere innerhalb von 5 Stunden, bevorzugt innerhalb von 30 min und besonders bevorzugt innerhalb von 10 min nach Entfernung des Porogens bestimmt.

Insbesondere wird die Porosität des erfindungsgemäßen Nanofaservlieses unmittelbar nach Entfernung des Porogens aus dem Nanofaservlies bestimmt, ohne dass das Nanofaservlies während des Bestimmens mechanisch belastet wird, beispielsweise durch Einspannen in eine Vorrichtung, insbesondere zum Beispiel eine Zellkulturvorrichtung. Die erfindungsgemäß bestimmte Porosität ist daher eine Porosität, die bevorzugt unmittelbar nach Entfernung des Porogens an einem keinen mechanischen Zug oder Druck oder sonstiger mechanischer Belastung ausgesetzten Nanofaservlies, insbesondere in der Flüssigkeit, insbesondere in Wasser, bestimmt wurde.

Bevorzugt wird die Größe der Masche in Form einer Maschenweite im erfindungsgemäßen Nanofaservlies ohne Porogen angegeben, insbesondere bestimmt mittels Mikroskopieverfahren. Die Maschenweite ist daher eine zweidimensionale Größe.

Die Messung der Maschenweite erfolgt nach Herauslösen des Porogens in Flüssigkeit, insbesondere in Wasser, insbesondere ohne mechanische Belastung des Nanofaservlieses, insbesondere ohne Einspannung des Nanofaservlieses, um die Struktur möglichst nicht zu beeinflussen. Am geeignetsten ist dabei die Anwendung von Laser-Scanning-Mikroskopie (LSM) oder der ähnlichen Methode konvokale Reflexionsmikroskopie. Aus dem entstandenen 3D-Bild wurden anschließend die Einzelbilder einer Nanofaserlage zu einem 2D-Bild übereinandergelegt. Anschließend wird die Fläche der Zwischenräume zwischen den Nanofasern bestimmt.

Im Zusammenhang mit der vorliegenden Erfindung wird unter einem "flexiblen Netzwerk" ein Netzwerk aus Nanofasern verstanden, in welchem Nanofasern und aus diesen Nanofasern gebildete Nanofaserlagen beweglich sind. Insbesondere bedeutet Flexibilität, dass Zellen in der Lage, sind einzelne Nanofasern, aber auch ganze Nanofaserlagen zu bewegen, insbesondere diese zusammenzuziehen.

In einer bevorzugten Ausführungsform sind die Nanofasern im Netzwerk aus Nanofasern entlang einer Ausdehnungsrichtung des Nanofaservlieses orientiert.

In einer bevorzugten Ausführungsform sind die Nanofasern im Netzwerk aus Nanofasern zufällig orientiert, das heißt insbesondere nicht entlang einer Ausdehnungsrichtung des Nanofaservlieses orientiert.

In einer bevorzugten Ausführungsform ist das Nanofasermaterial ausgewählt aus der Gruppe bestehend aus synthetischen Polymeren, insbesondere modifizierten natürlichen Polymeren, natürlichen Polymeren, zum Beispiel Chitosan, Hydroxyalkylcellulose, amorphen Metalloxiden und deren hybride (organisch/anorganisch) Modifikationen, Hybridmaterialien aus synthetischen und natürlichen Materialien, Keramik, Biogläser, Metalle, Kohlenstoff und Kombinationen davon.

In einer besonderen Ausführungsform der Erfindung ist das synthetische Polymer als Nanofasermaterial ausgewählt aus der Gruppe bestehend aus Polyester, insbesondere PCL, PLA, PGA, PET, PC sowie deren Copolymere, Polyamide, insbesondere Nylon- und Perlon-Typen, Polyurethane, Polyacrylnitril, Polystyrol, Polyethylen/Polypropylen, Polyvinylchlorid, Polyketone, insbesondere PEK, PEEK, Polyethylenglykole, Polyvinylalkohole, Polyoxazoline, Polypeptoide, Polypeptide, insbesondere Polylysin, und Kombinationen davon.

In einer besonderen Ausführungsform der Erfindung ist das natürliche Polymer als Nanofasermaterial ausgewählt aus der Gruppe bestehend aus Polysacchariden, insbesondere Chitosane, Cellulose-Derivate, insbesondere Hydroxyalkylcellulosen, Alginate, Lignine, Hyaluronsäure, Heparin, Strukturproteinen, insbesondere Kollagene, Fibrin, Elastin, Laminine, Vitronektin, Fibronektin, pflanzliche Proteine wie Zein, anderen Proteinen oder Peptiden, insbesondere Enzyme, Signalproteine, Wachstumsfaktoren, Nukleinsäuren, insbesondere DNA, RNA, und Kombinationen davon.

In einer besonderen Ausführungsform der Erfindung sind die Hybrid-Materialien als Nanofasermaterial Ormocer®e, Titan-Oxo-Carboxo-Cluster und/oder Silica- und Kieselgelmaterialien.

In einer besonderen Ausführungsform der Erfindung liegen Keramiken als Nanofasermaterial als Faser oder partikulär in Polymermatrix vor.

In einer besonderen Ausführungsform der Erfindung liegen Biogläser als Nanofasermaterial als Faser oder partikulär in Polymermatrix vor.

In einer besonderen Ausführungsform der Erfindung liegen Metalle als Nanofasermaterial partikulär in Polymermatrix vor.

In einer besonderen Ausführungsform der Erfindung liegt Kohlenstoff als Nanofasermaterial als Faser oder partikulär in Polymermatrix vor.

In einer besonders bevorzugten Ausführungsform weist das Nanofasermaterial eine Dichte von 0,5 bis 5 $g/cm^3$, insbesondere 1 bis 5 $g/cm^3$, insbesondere 1 bis 2,5 $g/cm^3$, insbesondere 1 bis 2 $g/cm^3$ auf.

In einer bevorzugten Ausführungsform weist das Nanofaservlies eine Dicke von 50 bis 5000 $\mu$m, insbesondere 100 bis 5000 $\mu$m, insbesondere 50 bis 4000 $\mu$m, insbesondere 100 bis 4000 $\mu$m, insbesondere 50 bis 3000 $\mu$m,

insbesondere 100 bis 3000 μm, insbesondere 50 bis 2000 μm, insbesondere 100 bis 2000 μm, insbesondere 50 bis 1000 μm, insbesondere 100 bis 1000 μm, insbesondere 50 bis 500 μm, insbesondere 100 bis 500 μm, auf.

In einer bevorzugten Ausführungsform umfasst das Porogen mindestens ein Porogenmaterial, insbesondere besteht aus diesem, ausgewählt aus der Gruppe bestehend aus wasserlöslichem Porogenmaterial, insbesondere Salze, Polymere und/oder Saccharide, Ethanol-löslichem Porogenmaterial, pH-sensitivem Porogenmaterial, enzymatisch abbaubarem Porogenmaterial, in physiologischen Lösungen degradierbare und/oder resorbierbare Porogene und Kombinationen davon.

In einer besonderen Ausführungsform der Erfindung ist das wasserlösliche Porogenmaterial ausgewählt aus der Gruppe bestehend aus wasserlöslichen Salzen, insbesondere NaCl, $NaCO_3$, wasserlöslichen Polymeren, insbesondere PEG/PEO, PVA, PVP, wasserlöslichen Sacchariden oder Polysacchariden, insbesondere Saccharose oder andere Zucker, Dextran, Hybridmaterialien, insbesondere Titan-Oxo-Carboxo-Cluster, und Kombinationen davon.

In einer besonders bevorzugten Ausführungsform ist das Porogenmaterial ausgewählt aus der Gruppe bestehend aus NaCl, $NaCO_3$, PEG/PEO, Zucker, $CaCO_3$, Chitosan, Zinkoxid und Kieselgel/amorphes Silica.

In einer besonders bevorzugten Ausführungsform ist das Porogenmaterial ausgewählt aus der Gruppe bestehend aus NaCl, $NaCO_3$, PEG/PEO und Zucker.

In einer besonders bevorzugten Ausführungsform ist das Porogenmaterial ausgewählt aus der Gruppe bestehend aus $CaCO_3$, Chitosan, Zinkoxid und Kieselgel/amorphes Silica.

In einer besonders bevorzugten Ausrufung der vorliegenden Erfindung liegt das Porogenmaterial partikulär vor.

In einer besonderen Ausführungsform der Erfindung ist das Ethanol-lösliche Porogenmaterial ein Polymer, insbesondere PVA.

In einer besonders bevorzugten Ausführungsform weist das Porogenmaterial eine Dichte von 0,01 bis 20 g/cm$^3$, insbesondere 0,5 bis 20 g/cm$^3$, insbesondere 1 bis 20 g/cm$^3$, insbesondere 5 bis 20 g/cm$^3$, insbesondere 0,5 bis 15 g/cm$^3$, insbesondere 1 bis 10 g/cm$^3$, insbesondere 2 bis 8 g/cm$^3$, insbesondere 1 bis 7 g/cm$^3$, bevorzugt 0,9 bis 5 g/cm$^3$, auf.

In einer besonderen Ausführungsform der vorliegenden Erfindung sind die Nanofasern im Netzwerk 2 bis 100 km lang.

In einer besonderen Ausführungsform der vorliegenden Erfindung sind die Nanofasern im Netzwerk so lang wie das Nanofaservlies.

Die Erfindung betrifft insbesondere auch ein zweites Verfahren zur Herstellung eines erfindungsgemäßen Nanofaservlieses, umfassend folgende Verfahrensschritte:

a') Bereitstellen mindestens eines Nanofasermaterials und mindestens eines zur Ausbildung mindestens eines Porogens geeigneten Porogenmaterials,

b') Elektroverspinnen des mindestens einen Nanofasermaterials unter Einbringen von aus dem mindestens einem Porogenmaterial ausgebildeten mindestens einem Porogen mit einem Durchmesser von 30 bis 1000 μm, so dass das Verhältnis von Porogenmaterial zu Nanofasermaterial in dem nach Abschluss dieses Verfahrensschrittes erhaltenen elektroversponnenen Nanofaservlies 95 bis 99 zu 5 bis 1 (jeweils Masse, bezogen auf die Gesamtmasse, also Porogene und Fasern, des Nanofaservlieses) beträgt und

c') Erhalten eines Nanofaservlieses umfassend mindestens ein Porogen, insbesondere eines Nanofaservlieses mit einer Porosität von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% (jeweils bezogen auf Gesamtvolumen des Nanofaservlieses).

In einer besonders bevorzugten Ausführungsform beträgt das Verhältnis von Porogenmaterial zu Nanofasermaterial in dem nach Abschluss des Verfahrensschrittes b') gemäß eines ersten Verfahrens erhaltenen elektroversponnenen Nanofaservlies 96 bis 99 zu 4 bis 1, insbesondere 97 bis 99 zu 3 bis 1, insbesondere 98 bis 99 zu 2 zu 1, insbesondere 95 bis 98 zu 5 zu 2, insbesondere 95 bis 97 zu 5 bis 3, insbesondere 95 bis 96 zu 5 bis 4, insbesondere 96 bis 98 zu 4 bis 2 (jeweils Masse, bezogen auf die Gesamtmasse, also Porogene und Nanofasermaterial, des Nanofaservlieses). Bevorzugt weisen so gemäß eines ersten und/oder zweiten Verfahrens hergestellte Nanofaservliese Maschen mit einer Weite von maximal 200 μm$^2$, insbesondere maximal 150 μm$^2$, insbesondere maximal 100 μm$^2$, insbesondere maximal 50 μm$^2$ (jeweils gemessen in Wasser ohne Porogen) auf.

Besonders bevorzugt weisen so hergestellte Nanofaservliese Maschen mit einer Weite von 10 bis 200 μm$^2$ (gemessen in Wasser ohne Porogen) auf. In einer besonders bevorzugten Ausführungsform weisen die gemäß einem erfindungsgemäßen Verfahren hergestellten Nanofaservliese Maschen mit einer Weite von 20 bis 200 μm$^2$, insbesondere 10 bis 150 μm$^2$, insbesondere 20 bis 150 μm$^2$, insbesondere 10 bis 100 μm$^2$, insbesondere 20 bis 100 μm$^2$, insbesondere 10 bis 50 μm$^2$, insbesondere 20 bis 50 μm$^2$, auf.

Die Erfindung betrifft auch vorgenannte erste und/oder zweite Verfahren zur Herstellung eines Nanofaservlieses, ferner umfassend die Verfahrensschritte:

d) Inkubieren des Nanofaservlieses erhalten in Verfahrensschritt c) oder c') umfassend mindestens ein Porogen in mindestens einem Lösungsmittel zum mindestens teilweisen Entfernen des mindestens einen Porogens aus dem Nanofaservlies und

e) Erhalten eines Nanofaservlieses.

Insbesondere betrifft die Erfindung auch Nanofaservliese hergestellt mit einem ersten und/oder zweiten Verfahren ferner umfassend die Verfahrensschritte d) und e).

In besonders bevorzugter Ausführungsform ist das Lösungsmittel zum Herauslösen des mindestens einen Porogens eine dazu geeignete Flüssigkeit, insbesondere eine Flüssigkeit, die die aus dem Nanofasermaterial gebildeten Nanofasern nicht angreift, insbesondere nicht abbaut, nicht strukturell ändert oder zerstört.

In besonders bevorzugter Ausführungsform ist die als Lösungsmittel verwendete Flüssigkeit Wasser. In besonders bevorzugter Ausführungsform ist diese Flüssigkeit eine wässrige Lösung, insbesondere einer oder mehrerer Substanzen In besonders bevorzugter Ausführungsform ist diese Flüssigkeit eine nicht-wässrige oder wässrige Lösung einer gegenüber dem Nanofasermaterial inerten Substanz oder Substanzgemisch in einem Lösungsmittel, zum Beispiel Wasser.

In besonders bevorzugter Ausführungsform weist das Lösungsmittel in Form einer wässrigen Lösung einen pH-Wert von 1 bis 13, insbesondere 1 bis 11, insbesondere 2 bis 11, insbesondere 3 bis 11, insbesondere 5 bis 9, vorzugsweise 6 bis 8, insbesondere 7, auf.

In einer besonders bevorzugten Ausführungsform, wenn zur Herstellung des Nanofaservlieses Calciumcarbonat als Porogenmaterial verwendet wurde, beträgt der pH-Wert des als wässrige Lösung ausgeführten Lösungsmittels 4 bis 5, insbesondere 4,5.

In einer besonders bevorzugten Ausführungsform, wenn zur Herstellung des Nanofaservlieses Chitosan als Porogenmaterial verwendet wurde, beträgt der pH-Wert des als wässrige Lösung ausgeführten Lösungsmittels 2 bis 4, insbesondere 3.

In einer besonders bevorzugten Ausführungsform, wenn zur Herstellung des Nanofaservlieses Zinkoxid als Porogenmaterial verwendet wurde, beträgt der pH-Wert des als wässrige Lösung ausgeführten Lösungsmittels 1 bis 3, insbesondere 2.

In einer besonders bevorzugten Ausführungsform, wenn zur Herstellung des Nanofaservlieses Kieselgel/amorphes Silica als Porogenmaterial verwendet wurde, beträgt der pH-Wert des als wässrige Lösung ausgeführten Lösungsmittels 11 bis 13, insbesondere 12.

In einer bevorzugten Ausführungsform wird nach dem Herauslösen der Porogene, insbesondere aller Porogene, das Lösungsmittel durch eine Flüssigkeit ersetzt, insbesondere durch eine wässrige Lösung, insbesondere Wasser.

Bevorzugt wird nach dem Herauslösen der Porogene, insbesondere aller Porogene, eine Flüssigkeit zu dem erfindungsgemäßen Nanofaservlies gegeben, insbesondere Wasser, so dass ein mindestens eine Flüssigkeit aufweisendes erfindungsgemäßes Nanofaservlies erhalten wird.

In bevorzugter Ausführungsform der vorliegende Erfindung wird nach dem Herauslösen aller oder eines Teils der Porogene dem Nanofaservlies mindestens eine Flüssigkeit, insbesondere Wasser, in einer Menge zugesetzt, dass in dem Nanofaservlies die mindestens eine Flüssigkeit und gegebenenfalls noch vorliegende Porogene mit einem Volumen von 90,0 bis 99,9 Vol.-%, insbesondere 92,5 bis 99,9 Vol.-%, insbesondere 95,0 bis 99,9 Vol.-%, insbesondere 98,5 bis 99,9 Vol.-% an Flüssigkeit und gegebenenfalls Porogenen (jeweils bezogen auf das Gesamtvolumen des Nanofaservlieses) vorliegen.

Die Erfindung betrifft auch ein vorgenanntes erstes und/oder zweites Verfahren zur Herstellung eines Nanofaservlieses, wobei das in Verfahrensschritt a) oder a') bereitgestellte Porogenmaterial partikelförmig oder faserförmig ist, einen Durchmesser von 30 bis 1000 $\mu$m, insbesondere 40 bis 1000 $\mu$m, insbesondere 50 bis 1000 $\mu$m, insbesondere 70 bis 1000 $\mu$m, insbesondere 100 bis 1000 $\mu$m, insbesondere 30 bis 500 $\mu$m, insbesondere 40 bis 500 $\mu$m, insbesondere 50 bis 500 $\mu$m, insbesondere 70 bis 500 $\mu$m, insbesondere 100 bis 500 $\mu$m, insbesondere 30 bis 250 $\mu$m, insbesondere 40 bis 250 $\mu$m, insbesondere 50 bis 250 $\mu$m, insbesondere 70 bis 250 $\mu$m, insbesondere 100 bis 250 $\mu$m, insbesondere 30 bis 150 $\mu$m, insbesondere 40 bis 150 $\mu$m, insbesondere 50 bis 150 $\mu$m, insbesondere 70 bis 150 $\mu$m, insbesondere 100 bis 150 $\mu$m, aufweist und in Verfahrensschritt a) oder a') als ausgebildetes Porogen bereitgestellt wird.

In einer besonders bevorzugten Ausführungsform weist das in Verfahrensschritt a) oder a') bereitgestellte Porogenmaterial einen Durchmesser von 30 bis 1000 $\mu$m, insbesondere 40 bis 1000 $\mu$m, insbesondere 50 bis 1000 $\mu$m, insbesondere 70 bis 1000 $\mu$m, insbesondere 100 bis 1000 $\mu$m, insbesondere 30 bis 500 $\mu$m, insbesondere 40 bis 500 $\mu$m, insbesondere 50 bis 500 $\mu$m, insbesondere 70 bis 500 $\mu$m, insbesondere 100 bis 500 $\mu$m, insbesondere 30 bis 250 $\mu$m, insbesondere 40 bis 250 $\mu$m, insbesondere 50 bis 250 $\mu$m, insbesondere 70 bis 250 $\mu$m, insbesondere 100 bis 250 $\mu$m, insbesondere 30 bis 150 $\mu$m, insbesondere 40 bis 150 $\mu$m, insbesondere 50 bis 150 $\mu$m, insbesondere 70 bis 150 $\mu$m, insbesondere 100 bis 150 $\mu$m, auf, wobei mindestens 70 %, insbesondere mindestens 80 %, insbesondere mindestens 90 %, insbesondere mindestens 95 %, insbesondere mindestens 99 %, insbesondere 100 %, des in Verfahrensschritt a) oder a') bereitgestellten Porogenmaterials einen Durchmesser von mindestens 30 $\mu$m, insbesondere mindestens 40 $\mu$m, insbesondere mindestens 50 $\mu$m, insbesondere mindestens 70 $\mu$m, insbesondere mindestens 100 $\mu$m, aufweist.

In einer besonders bevorzugten Ausführungsform weist mindestens 70 %, insbesondere mindestens 80 %, insbesondere mindestens 90 %, insbesondere mindestens 95 %, insbesondere mindestens 99 %, insbesondere 100 %, des in Verfahrensschritt a) oder a') bereitgestellten Porogenmaterials einen Durchmesser von 30 bis 1000 $\mu$m, insbesondere 40 bis 1000 $\mu$m, insbesondere 50 bis 1000 $\mu$m, insbesondere 70 bis 1000 $\mu$m, insbesondere 100 bis 1000 $\mu$m, insbesondere 30 bis 500 $\mu$m, insbesondere 40 bis 500 $\mu$m, insbesondere 50 bis 500 $\mu$m, insbesondere 70 bis 500 $\mu$m, insbesondere 100 bis 500 $\mu$m, insbesondere 30 bis 250 $\mu$m, insbesondere 40 bis 250 $\mu$m, insbesondere 50 bis 250 $\mu$m, insbesondere 70 bis 250 $\mu$m, insbesondere 100 bis 250 $\mu$m, insbesondere 30 bis 150 $\mu$m, insbesondere 40 bis 150 $\mu$m, insbesondere 50 bis 150 $\mu$m, insbesondere 70 bis 150 $\mu$m, insbesondere 100 bis 150 $\mu$m, auf.

Das Porogen wird in Verfahrensschritt

a) oder a') als fertig ausgebildetes Porogen bereitgestellt und in Verfahrensschritt b) oder
b') wird alternierend ein Einbringen des Porogens und das Elektroverspinnen des Nanofasermaterials durchgeführt.

Alternativ dazu ist das in Verfahrensschritt a) oder a') bereitgestellte Porogenmaterial ein zum Ausbilden mindestens eines faserförmigen Porogens geeignetes Material. Im Verfahrensschritt b) oder b') wird aus dem Porogenmaterial vor oder während dessen Einbringen in das elektroversponnene Nanofasermaterial mindestens ein faserförmiges Porogen ausgebildet, insbesondere kontinuierlich, insbesondere kontinuierlich und gleichzeitig.

In einer besonders bevorzugten Ausführungsform wird das Porogenmaterial in Verfahrensschritt b) oder b') mittels Druckspinnverfahren zu einem oder mehreren faserförmigen Porogenen ausgebildet, in besonders bevorzugter Ausführungsform während des Einbringens des Porogenmaterials in das elektroversponnene Nanofasermaterial in Verfahrensschritt b) oder b').

In einer bevorzugten Ausführungsform wird in Verfahrensschritt b) oder b') ein faserförmiges Porogen durch Abrollen von einer Spindel auf das elektroversponnene Nanofasermaterial aufgebracht.

In einer bevorzugten Ausführungsform wird das Elektroverspinnen in Verfahrensschritt b) oder b') mit einer geerdeten rotierenden Walze oder einer geerdeten Platte durchgeführt.

In einer bevorzugten Ausführungsform wird das Porogenmaterial mittels 3D-Druckverfahren, insbesondere Fused Deposition Modeling (FDM), Bioprinting/Bioplotting, Selective Laser Scintering oder Melt Electro Writing (MEW), in Verfahrensschritt b) oder b') auf das elektroversponnene Nanofasermaterial aufgetragen.

In einer bevorzugten Ausführungsform wird das Porogenmaterial mittels Ink-Jet-Verfahren in Verfahrensschritt b) oder b') auf das elektroversponnene Nanofasermaterial aufgetragen.

In einer bevorzugten Ausführungsform wird die Haftkraft des mindestens einen Porogenmaterials vor dem Einbringen in Verfahrensschritt b) oder b') erhöht, bevorzugt durch eine teilweise Behandlung der Oberfläche des Porogenmaterials mit einem Lösungsmittel, in welchem das Porogenmaterial sich nur wenig löst, insbesondere mit einer Löslichkeit von maximal 0,5 g Porogenmaterial/100g Lösungsmittel, insbesondere maximal 0,2 g Porogenmaterial/100 g Lösungsmittel, insbesondere genau 0,065g Porogenmaterial/100g Lösungsmittel jeweils bei 25 °C, wobei das Porogenmaterial in bevorzugterweise partikelförmig oder faserförmig, insbesondere partikelförmig, vorliegt.

In einer besonders bevorzugten Ausführungsform der vorliegenden Erfindung wird in Verfahrensschritt b) oder b') das Elektroverspinnen mit Fluggeschwindigkeiten von 1 bis 100 m/s insbesondere 5 bis 50 m/s, insbesondere 8 bis 30 m/s durchgeführt.

In einer bevorzugten Ausführungsform der vorliegenden Erfindung wird das Elektroverspinnen in Verfahrensschritt b) oder b') kontinuierlich, insbesondere kontinuierlich und gleichzeitig, durchgeführt.

In einer bevorzugten Ausführungsform der vorliegenden Erfindung wird das Elektroverspinnen in Verfahrensschritt b) oder b') alternierend durchgeführt.

In einer besonderen Ausführungsform der vorliegenden Erfindung wird das in Verfahrensschritt c) oder c') erhaltene Nanofaservlies anschließend mechanisch bearbeitet, insbesondere zerschnitten. Die vorliegende Erfindung betrifft auch Nanofaservliese, herstellbar oder hergestellt gemäß einem der erfindungsgemäßen Verfahren, insbesondere des ersten oder zweiten Verfahrens.

Auch solche Nanofaservliese werden in bevorzugter Ausführungsform als erfindungsgemäße Nanofaservliese bezeichnet.

Die Erfindung betrifft auch die Verwendung eines erfindungsgemäßen Nanofaservlieses zur Kultivierung oder Differenzierung von Zellen.

Die Erfindung betrifft auch ein künstliches Gewebe umfassend mindestens ein erfindungsgemäßes Nanofaservlies und mindestens eine Zelle mindestens eines Zelltyps, optional zusammen mit extrazellulärer Matrix. Die Erfindung betrifft auch ein künstliches Gewebe umfassend mindestens ein erfindungsgemäßes Nanofaservlies und Zellen mindestens eines Zelltyps, optional zusammen mit extrazellulärer Matrix.

In einer bevorzugten Ausführungsform der Erfindung ist der mindestens eine Zelltyp ausgewählt aus der Gruppe bestehend aus Fibroblasten, insbesondere cancer associated fibroblasts, Keratinozyten, mesenchymale Stammzellen, iPS-Zellen (induziert pluripotente Stammzellen), Tumorzellen, Endothelzellen und Kombinationen davon.

In einer bevorzugten Ausführungsform weist das künstliche Gewebe mehrere, insbesondere mindestens zwei, insbesondere mindestens drei, erfindungsgemäße Nanofaservliese auf.

Die Erfindung betrifft auch ein künstliches Gewebe, umfassend ein erfindungsgemäßes Nanofaservlies und Zellen mindestens eines Zelltyps, wobei das künstliche Gewebe 1 bis 50 Gew.-% Nanofaservlies und 50 bis 99 Gew.-% Zellen, insbesondere 80 bis 99 Gew.-% Zellen und, optional, extrazelluläre Matrix, insbesondere 1 bis 49 Gew.-% extrazelluläre Matrix, umfasst (jeweils bezogen auf das Gesamtgewicht des künstlichen Gewebes).

Die Erfindung betrifft auch ein künstliches Gewebe umfassend ein erfindungsgemäßes Nanofaservlies und Zellen mindestens eines Zelltyps, wobei das künstliche Gewebe 1 bis 50 Gew.-% Nanofaservlies und 50 bis 99 Gew.-% Zellen (jeweils bezogen auf das Gesamtgewicht des künstlichen Gewebes) aufweist.

Die Erfindung betrifft auch ein künstliches Gewebe umfassend ein erfindungsgemäßes Nanofaservlies und Zellen mindestens eines Zelltyps, wobei das künstliche Gewebe 1 bis 49 Gew.-% Nanofaservlies und 50 bis 99 Gew.-% Zellen und 1 bis 49 Gew.-% extrazelluläre Matrix umfasst (jeweils bezogen auf das Gesamtgewicht des künstlichen Gewebes) aufweist.

Die Erfindung betrifft auch ein künstliches Gewebe, wobei das im Nanofaservlies vorliegende mindestens eine Porogen vor Besiedelung mit Zellen aus dem Nanofaservlies mindestens teilweise entfernt wurde.

In bevorzugter Ausführungsform ist das künstliche Gewebe ein Hautmodell, insbesondere umfassend eine Dermis, umfassend mindestens ein erfindungsgemäßes Nanofaservlies. In bevorzugter Ausführungsform ist das künstliche Gewebe ein Hautmodell, bevorzugt umfassend eine Subcutis umfassend mindestens ein erfindungsgemäßes Nanofaservlies.

In bevorzugter Ausführungsform ist das künstliche Gewebe ein Hautmodell, insbesondere umfassend eine Dermis, umfassend mindestens ein erfindungsgemäßes Nanofaservlies und eine Subcutis umfassend mindestens ein erfindungsgemäßes Nanofaservlies, vorzugsweise ein unterschiedliches Nanofaservlies.

Bevorzugt sind die in dem erfindungsgemäßen Hautmodell eingesetzten mindestens zwei Nanofaservliese spezifisch ausgebildet, insbesondere weisen sie jeweils mindestens eine Zelle eines spezifischen Zelltyps auf, insbesondere unterschiedlichen Zelltyps.

Die Erfindung betrifft auch ein Verfahren zur Herstellung eines künstlichen Gewebes umfassend folgende Verfahrensschritte:

x1) Bereitstellen von mindestens einer Zelle mindestens eines Zelltyps, einem Kulturmedium und mindestens einem erfindungsgemäßen Nanofaservlies,

x2) optionales Entfernen mindestens eines Teils eines gegebenenfalls vorhandenen mindestens eines Porogens,

x3) Kultivieren des mindestens einen Nanofaservlieses mit der mindestens einen Zelle in dem Kulturmedium und

x4) Erhalten eines künstlichen Gewebes umfassend mindestens eine auf oder in dem mindestens einen Nanofaservlies angeordnete Zelle.

In bevorzugter Ausführungsform der vorliegenden Erfindung erfolgt während des in Verfahrensschritt x3) vorgesehenen Kultivierens eine Besiedlung des erfindungsgemäßen Nanofaservlieses durch die Maschen des Nanofaservlieses in die Poren des Nanofaservlieses, wobei vorzugsweise und optional anschließend ein Zusammenziehen der Poren zu einem dreidimensionalen erfindungsgemäßen künstlichen Gewebe führt, das aus Nanofasern, Zellen und von diesen gebildeten Proteinen umfasst.

In bevorzugter Ausführungsform liegen die Zellen homogen verteilt innerhalb des erfindungsgemäßen Nanofaservlieses vor und führen in weiterer bevorzugter Ausführungsform zu einer homogenen Verteilung der extrazellulären Matrix, insbesondere der Proteine.

Die Erfindung betrifft auch ein derartiges Verfahren, wobei während oder nach der Kultivierung gemäß Verfahrensschritt x3) das Nanofaservlies in einem Verfahrensschritt x31) mit mindestens einer Zelle mindestens eines weiteren Zelltyps, insbesondere zu dem in Verfahrensschritt x1) bereitgestellten Zelltyp unterschiedlichen Zelltyps, kultiviert wird.

Die Erfindung betrifft auch ein derartiges Verfahren, wobei während oder nach der Kultivierung gemäß Verfahrensschritt x3) oder x31) weitere Nanofaservliese, bevorzugt besiedelt mit Zellen von gleichem oder anderem Zelltyp, auf das Nanofaservlies, erhalten in x3) oder x31), gestapelt werden.

In einer bevorzugten Ausführungsform der Erfindung werden in Verfahrensschritt x1) im Verfahren zur Herstellung eines künstlichen Gewebes 20.000 bis 150.000 Zellen/cm$^2$, insbesondere 40.000 bis 100.000 Zellen/cm$^2$, des

mindestens einen Zelltyps bereitgestellt.

In einer bevorzugten Ausführungsform der Erfindung wird das mindestens eine erfindungsgemäße Nanofaservlies in einem Verfahrensschritt x21) eingespannt in eine Zellkrone oder Transwell®-Insert.

Im Zusammenhang mit der vorliegenden Erfindung wird unter einer "Zellkrone" zwei unterschiedlich große Ringe, bevorzugt Edelstahlringe, verstanden, welche so ineinander steckbar sind, dass zwischen diesen ein Nanofaservlies teilweise eingespannt wird und so ein Teil des Nanofaservlieses über den freien Raum des kleineren Ringes gespannt ist.

Die vorliegende Erfindung betrifft auch therapeutische Anwendungen der erfindungsgemäßen Nanofaservliese und diese aufweisenden künstlichen Gewebe.

Diese Anwendungen betreffen die Therapie von erkrankten, Menschen oder Tieren.

Insbesondere kann das erfindungsgemäße Nanofaservlies als Wundeinlage, insbesondere zur Therapie von bevorzugt chronischen Wunden oder Brandwunden, insbesondere unter Einbringen, insbesondere Einlegen, des hochporösen erfindungsgemäßen Nanofaservlieses in Wunden, Besiedelung mit Gewebezellen aus den Wundrändern, Verschließung der Wunde über dem erfindungsgemäßen Nanofaservlies durch Zellen, insbesondere Keratinozyten eingesetzt werden. Das erfindungsgemäße Nanofaservlies kann auch als poröses Gewebe, welches in-vivo das Einwachsen von Zellen während der Regeneration nach zum Beispiel chirurgischen Eingriffen oder Resektionen in der (Krebs-)therapie ermöglicht, verwendet werden.

Insbesondere kann das erfindungsgemäße künstliche Gewebe als ATMP (Advanced Therapy Medicinal Product) eingesetzt werden, in bevorzugter Ausführungsform besiedelt mit gewebespezifischen Zellen, wobei der Einsatz in oder als stromalen bzw stromales Gewebe/Organ(en) bevorzugt ist, insbesondere in oder als Haut, Knorpel, Arterien und/oder Venen, Herz, Niere, Leber, Urogenitaltrakt, Atemwege, Knochen, Darm oder Cornea.

Die vorliegende Erfindung betrifft auch nicht-therapeutische Anwendungen der erfindungsgemäßen Nanofaservliese und diese aufweisenden künstlichen Gewebe, zum Beispiel kosmetische Anwendungen.

Im Zusammenhang mit der vorliegenden Erfindung wird das Volumenverhältnis von Porogenmaterial zu Nanofasermaterial in dem nach Abschluss des Elektroverspinnens erhaltenen elektroversponnenen Nanofaservlies bestimmt durch Bestimmung der Volumina des im erhaltenen elektroversponnenen Nanofaservlieses vorhandenen Porogenmaterials und Nanofasermaterials. Die Volumina des im erhaltenen elektroversponnenen Nanofaservlies enthaltenen Porogenmaterials und Nanofasermaterials lässt sich ermitteln durch Bestimmung der Masse des Porogenmaterials und Nanofasermaterials, welche sich in dem erhaltenen elektroversponnenen Nanofaservlies befindet, wobei unter Berücksichtigung der bekannten Dichte des im erhaltenen elektroversponnenen Nanofaservlieses vorhandenen Porogenmaterials und Nanofasermaterial das Volumen ermittelt werden kann.

Im Zusammenhang mit der vorliegenden Erfindung wird unter dem Begriff "und/oder" verstanden, dass alle Mitglieder einer Gruppe, welche durch den Begriff "und/oder" verbunden sind, sowohl untereinander kumulativ in einer beliebigen Kombination, als auch alternativ zueinander dargestellt sind. Exemplarisch ist für den Ausdruck "A, B und/oder C", folgender Offenbarungsgehalt darunter zu verstehen: i) (A oder B oder C), oder ii) (A und B), oder iii) (A und C), oder iv) (B und C), oder v) (A und B und C), oder vi) (A und B oder C), oder vii) (A oder B und C), oder viii) (A und C oder B).

Im Zusammenhang mit der vorliegenden Erfindung addieren Einzelkomponenten oder Bestandteile einer Gesamtheit, insbesondere des erfindungsgemäßen Nanofaservlieses oder einer seiner Komponenten, die quantitativ in relativer Form, insbesondere in Prozentangaben bestimmt sind, sofern nicht anders angegeben, vorzugsweise auf 100 Gew.-% der jeweils in Bezug genommenen Gesamtheit beziehungsweise des Nanofaservlieses oder, sofern in Bezug genommen, einer Komponente davon, auf.

Im Zusammenhang mit der vorliegenden Erfindung werden, sofern nicht anders angegeben, nicht angegebene Nachkommastellen einer Dezimalzahl als 0 verstanden.

Bezugszeichenliste:

1    z-Richtung, auch als Höhe oder Dicke bezeichnet

2    x-Richtung, auch als Breite bezeichnet

3    y-Richtung, auch als Tiefe bezeichnet

4    Nanofaserlagen

5    Poren

6       Porogene, insbesondere lösliche Porogene

7       Nanofasern

8       Maschen, schraffierte Fläche bezeichnet die Maschenweite

9       Porenbreite

10      Porentiefe

11      Porenhöhe

12      Nanofaservliesdicke/Nanofaservlieshöhe

13      Nanofaservliesbreite (eine Haupt(Flächen-)ausdehnungsrichtung des Nanofaservlieses)

14      Nanofaservliestiefe (eine Haupt(Flächen-)ausdehnungsrichtung des Nanofaservlieses)

100     Erfindungsgemäßes Nanofaservlies

Weitere vorteilhafte Ausgestaltungen der Erfindung ergeben sich aus den Unteransprüchen.
Die nachfolgenden Beispiele und die dazugehörigen Figuren erläutern die vorliegende Erfindung, ohne sie jedoch einzuschränken.
Die Figuren zeigen:

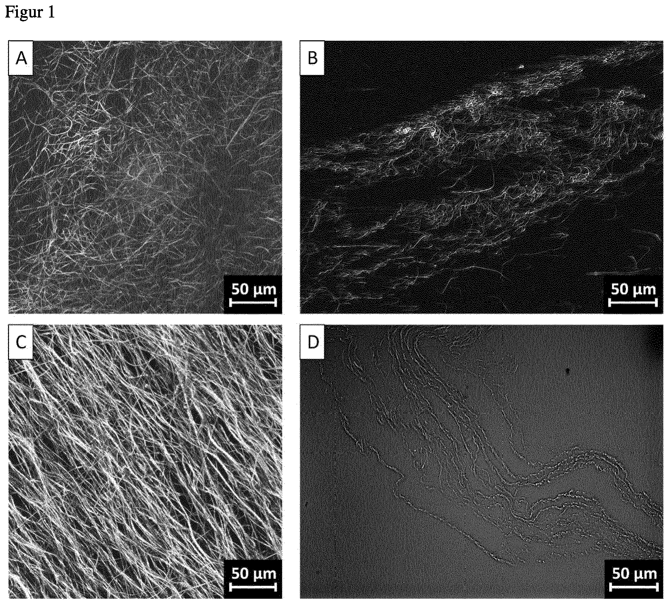

Figur 1      Beispiele für Strukturen von erfindungsgemäßen hochporösen Nanofaservliesen. (A) Hochporöses Nanofaservlies mit zufällig orientierten Nanofasern (7), aufgenommen in Wasser durch konfokale Reflexionsmikroskopie. (B) Poröse Struktur des zufällig orientierten Nanofaservlies, präpariert durch einen Paraffinschnitt und aufgenommen über ein Lichtmikroskop mit anschließender Bildbearbeitung. (C) Hochporöses Nanofaservlies mit orientierten Nanofasern (7), aufgenommen wie (A). (D) Poröse Struktur des orientierten Nanofaservlies, präpariert und aufgenommen wie (B).

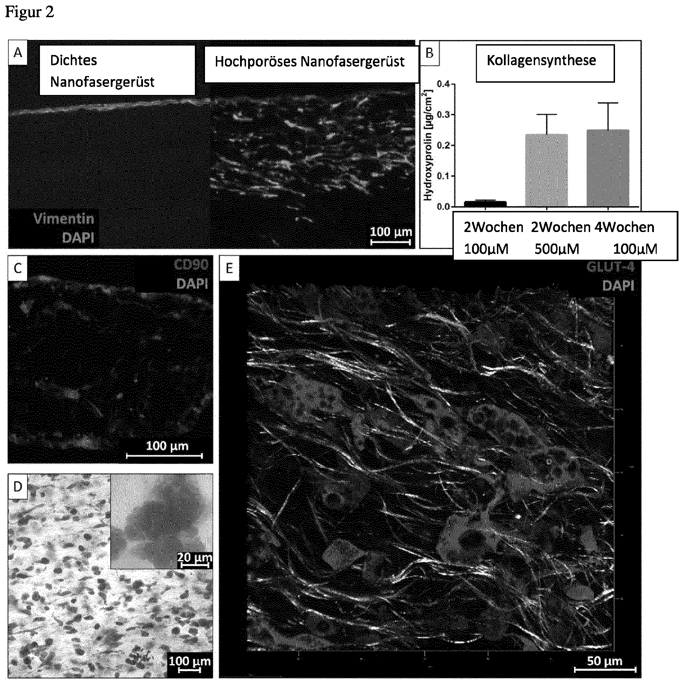

Figur 2      Eigenschaften der erfindungsgemäßen hochporösen Nanofaservliese in Bezug auf biologische Prozesse. (A) Im Vergleich zu einem dichten nicht-erfindungsgemäßen Nanofaservlies können Fibroblasten das gesamte hochporöse Nanofaservlies selbstständig besiedeln. (B) Während der Besiedelung sind diese Zellen in der Lage, Gewebe wie Kollagen innerhalb des Nanofaservlieses zu synthetisieren. (C) Diese Migrationseigenschaften im hochporösen Nanofaservlies sind für alle Typen an Gewebezellen gegeben, wie zum Beispiel. für MSCs. (D, E) Speziell MSCs können innerhalb des Nanofaservlieses differenziert werden, beispielsweise in die adipogene Richtung.

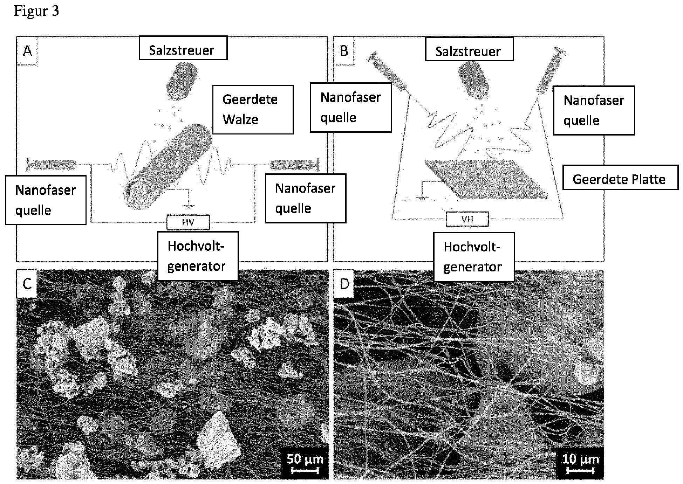

Figur 3      Herstellung von erfindungsgemäßen hochporösen Nanofaservliesen mit Partikeln (A) Schematische Zeichnung des Spinnaufbaus mit rotierendem zylindrischen Kollektor, Partikelzugabe und horizontal gegenüberliegenden Nanofaserquellen. (B) Schematischer Aufbau mit Kollektorplatte statt Zylinder. (C) REM-Aufnahme von NaCl-Partikeln innerhalb des Nanofaservlieses, (D) sowie Nahaufnahme der Nanofasern (7).

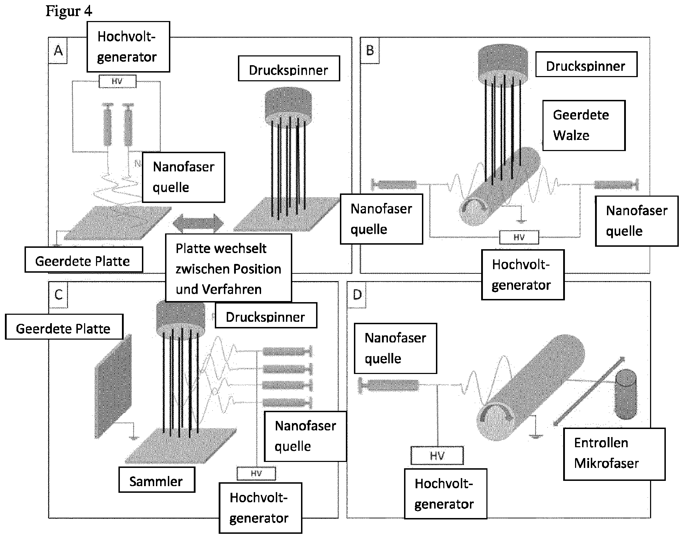

Figur 4      Herstellung von erfindungsgemäßen hochporösen Nanofaservliesen in Kombination mit Fasern im Mikrometerbereich. (A) Schematische Zeichnung der Kombination Elektro-Spinnen/Druckspinnen alternierend auf eine Platte. (B) Schematische Darstellung der Kombination Elektro-Spinnen/Druckspinnen gleichzeitig auf einen rotierenden Zylinder. (C) Schematischer Aufbau der Kombination Elektro-Spinnen/Druckspinnen, wobei die Nanofasern (7) direkt in den Lauf der Mikrofasern gesponnen werden. (D) Schematische Zeichnung der Kombination Elektro-Spinnen mit einer bereits hergestellten Faser, welche während des E-Spinnvorgangs kontinuierlich zwischen die Nanofasern (7) auf den rotierenden Zylinder gewickelt werden.

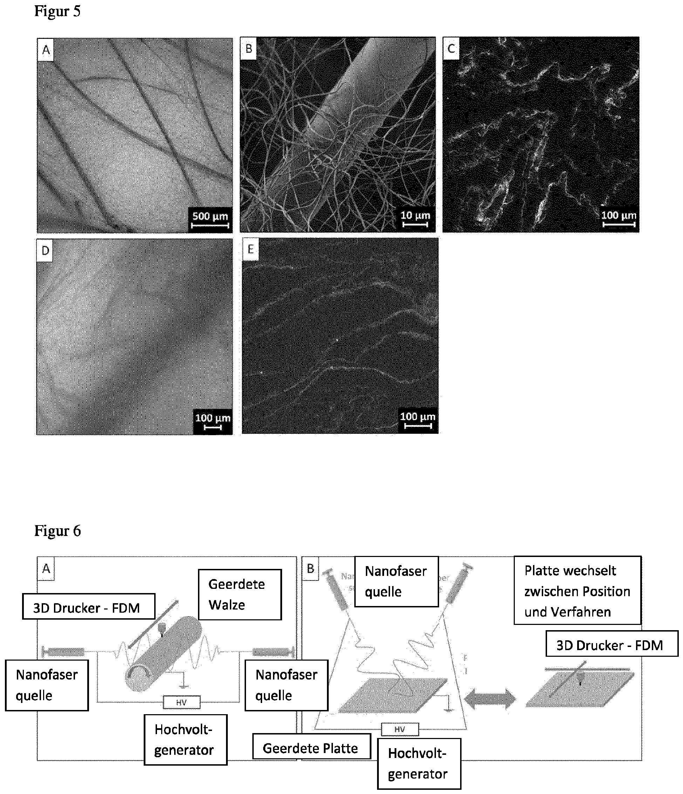

Figur 5      Mikroskopie der Faserstruktur durch die Kombination von Elektrospinnen mit Mikrofasern. (A) Lichtmikroskopische Aufnahme von Druck-gesponnenen Fasern und den kombinierten Nanofasern (7) (Methode: Abbildung 4A). (B) REM-Aufnahme zeigt den Dimensionsunterschied der beiden Fasern. (C) Nach Herauslösen der Mikrofasern beschreibt der Querschnitt eine hohe Porosität. (D) Lichtmikroskopische Aufnahme von Nanofasern (7), kombiniert mit einem gleichzeitig aufgewickeltem Faden im Mikrometerbereich (Methode: Abbildung 4D). (E) Nach Herauslösen der Mikrofasern beschreibt der Querschnitt eine hohe Porosität.

Figur 6    Herstellung von erfindungsgemäßen hochporösen Nanofaservliesen in Kombination mit FDM oder MEW. (A) Schematische Darstellung der Kombination E-Spinnen/3D-Druck gleichzeitig auf einen rotierenden Zylinder. (B) Schematische Zeichnung der Kombination E-Spinnen/Druckspinnen alternierend auf eine Platte.

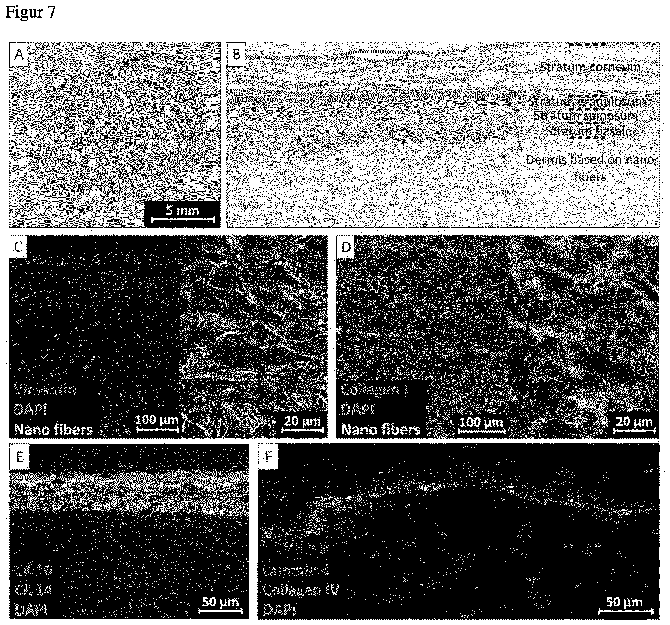

Figur 7    Anwendungsbeispiel von erfindungsgemäßen hochporösen Nanofaservliesen in der 3D Gewebekultur - Haut aus Demis und Epidermis. (A) Foto eines in vitro generierten Hautmodells. (B) HE-Färbung des Querschnittes beschreibt die einzelnen physiologischen Schichten der Epidermis, sowie teilweise die Nanofaserbasierte Dermis. (B) IF-Färbung gegen Vimentin zeigt die homogene Verteilung der Fibroblasten im dermalen hochporösen Faservlies. Der rechte Teil zeigt in einer höheren Vergrößerung gleichzeitig die Fasern und die Fibroblasten. (D) IF-Färbung gegen Kollagen I beschreibt die Verteilung der synthetisierten EZM. Der rechte Teil zeigt in einer höheren Vergrößerung gleichzeitig die Fasern und die biologische Matrix. (E) IF-Färbungen gegen Cytokeratin 10 und 14, sowie gegen (D) Laminin und Collagen 4 weisen die nachgebildeten physiologischen Bereiche der Epidermis nach.

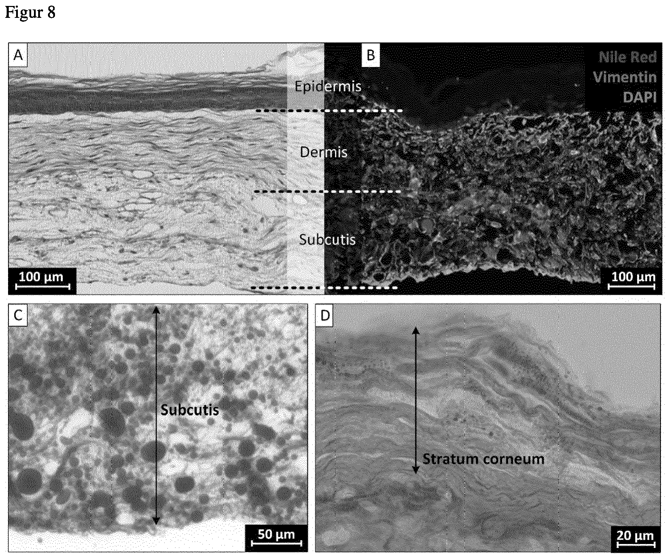

Figur 8    Anwendungsbeispiel von erfindungsgemäßen hochporösen Nanofaservliesen in der 3D Gewebekultur - Haut aus Subcutis, Demis und Epidermis. (A) HE-Färbung des Querschnittes beschrieb die einzelnen physiologischen Schichten des dreischichtigen Hautmodells. Die leeren Bläschen in der Subcutis stellen die adipogenen Zellen dar. (B) IF-Färbung gegen Vimentin mit Nile Red zeigt die homogene Verteilung der Fibroblasten im hochporösen Faservlies, sowie die Fetttröpfchen im tieferen Teil des Modells. (E) Darstellung der Subcutis durch Färbung der Fetttröpfchen mit Öl-Rot. (D) Neben der Subcutis können ebenfalls nanoskalierte Fetttröpfchen innerhalb der Epidermis angefärbt und abgelichtet werden.

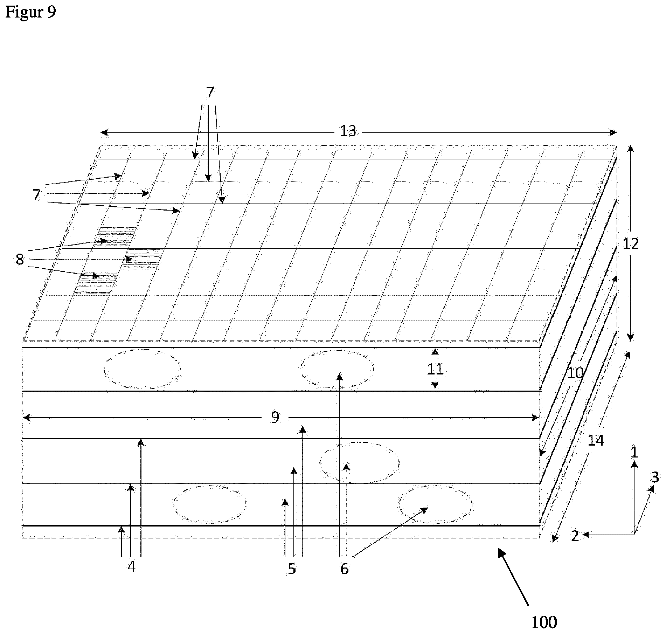

Figur 9    Schematische Zeichnung eines erfindungsgemäßen Nanofaservlieses zur Erläuterung des Zusammenhangs zwischen den einzelnen strukturellen Bestandteilen eines erfindungsgemäßen Nanofaservlieses. Dargestellt ist ein erfindungsgemäßes Nanofaservlies 100, dessen Ausdehnung in z-Richtung 1 sowie den Flächenausdehnungsrichtungen 2 und 3 in x- und y-Richtung. Dargestellt sind die Nanofaserlagern 4, die Poren 5, die Porogene 6, die Nanofasern 7 und die durch die Nanofasern gebildeten Maschen 8. Dargestellt ist auch die Porenbreite 9, Porentiefe 10 und Porenhöhe 11 sowie die Nanofaservliesdicke beziehungsweise -höhe 12, die Nanofaservliesbreite, nämlich eine Hauptausdehnungsrichtung des Nanofaservlieses 13 sowie die Nanofaservliestiefe, nämlich eine weitere Hauptausdehnungsrichtung des Nanofaservlieses 14.

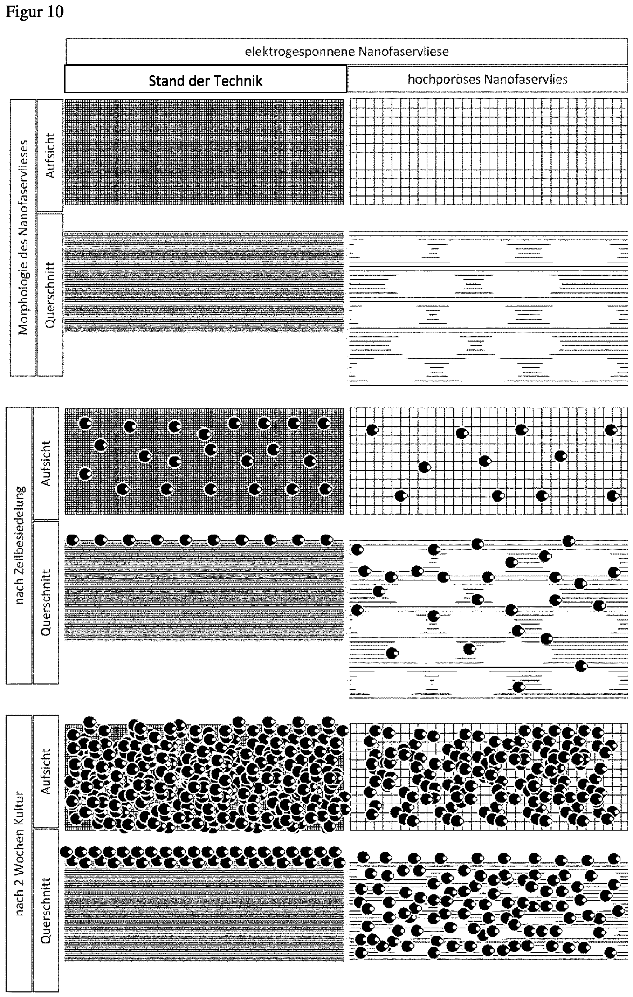

Figur 10    schematische Zeichnung der Besiedelung eines nicht-erfindungsgemäßen Nanofaservlieses (Stand der Technik) (linke Seite) und eines hochporösen erfindungsgemäßen Nanofaservlieses (rechte Seite) mit Zellen (schwarze Punkte mit kleinerem weißem Punkt).
Teil 1: Morphologie des Nanofaservlieses
Bei Betrachtung der Nanofaservliese von oben weist das hochporöse erfindungsgemäße Nanofaservlies wesentlich größere Freiräume zwischen den Nanofasern (7) auf (bezeichnet als Maschen (8)). Während diese bei dichten nicht-erfindungsgemäßen Nanofaservliesen auf etwa 5 $\mu m^2$ begrenzt sind, können die Maschen der hochporösen erfindungsgemäßen Nanofaservliese Durchtrittsflächen von über 100 $\mu m^2$ erreichen. Im Querschnitt sind Nanofasern (7) im dichten nicht-erfindungsgemäßen Nanofaservlies direkt übereinander abgelegt. Das hochporöse erfindungsgemäße Nanofaservlies besitzt dagegen Poren (5) innerhalb des Nanofaservlieses, welche durch Porogene (6) erzeugt werden. Diese Poren (5) können seitlich abgeschlossen sein, können aber auch das gesamte Nanofaservlies in der Flächenausdehnungsrichtung (13, 14) durchdringen. Des Weiteren liegen die Nanofasern (7) im hochporösen erfindungsgemäßen Nanofaservlies nicht immer im direkten Kontakt übereinander, weswegen der Abstand zwischen den Nanofasern (7) teilweise erhöht ist.
Teil 2: Nach Zellbesiedelung
Werden Zellen auf das dichte nicht-erfindungsgemäße Nanofaservlies gegeben, bleiben diese auf der Oberfläche des nicht-erfindungsgemäßen Nanofaservlieses und vermehren sich dort. Im Falle des hochporösen erfindungsgemäßen Nanofaservlieses können die Zellen das Nanofaservlies von oben nach unten komplett durchdringen. Dadurch verweilen wesentlich weniger Zellen auf der Oberfläche des hochporösen Nanofaservlieses, können sich aber im Nanofaservlies stark vermehren. Die Zellen sind dabei in der Lage, sich senkrecht (Dicke/Höhe des Nanofaservlieses, 12) durch das Nanofaservlies über die vergrößerten Maschen (8) zu bewegen. In der Breite des Nanofaservlieses (13) können sich die Zellen entweder an den Oberflächen der Poren (5) oder durch die erweiterten Abstände zwischen den Nanofasern (7) bewegen. Teil 3: nach 2 Wochen Kultur

Die Zellen auf dem dichten nicht-erfindungsgemäßen Nanofaservlies haben nach 2 Wochen Kultur die komplette Oberfläche besiedelt und können (je nach Zelltyp) übereinander weiter proliferieren, dringen aber nicht in das Nanofaservlies ein. Im hochporösen erfindungsgemäßen Nanofaservlies vermehren sich die Zellen hauptsächlich innerhalb des Vlieses. Dabei interagieren die Zellen mit dem Nanofaservlies und ziehen das hochflexible Nanofaservlies zusammen. Dadurch vermehren sich die Zellen innerhalb der Poren (5) nicht direkt übereinander wie auf der Oberfläche des dichten nicht-erfindungsgemäßen Nanofaservlieses. Die Kontraktion des hochporösen erfindungsgemäßen Nanofaservlieses durch die Zellen führt dazu, dass die Porenhöhe (11) im Nanofaservlies kontinuierlich sinkt bis diese nicht mehr erkennbar sind. Dadurch wandeln die Zellen das Nanofaservlies derart um, das eine nahezu homogene Verteilung von Zellen und Nanofasern (7) in alle drei Raumrichtungen entsteht. Nach Erreichen einer maximalen Zelldichte reduziert sich die Proliferation innerhalb des Nanofaservlieses und findet hauptsächlich auf oder unter dem Nanofaservlies statt.

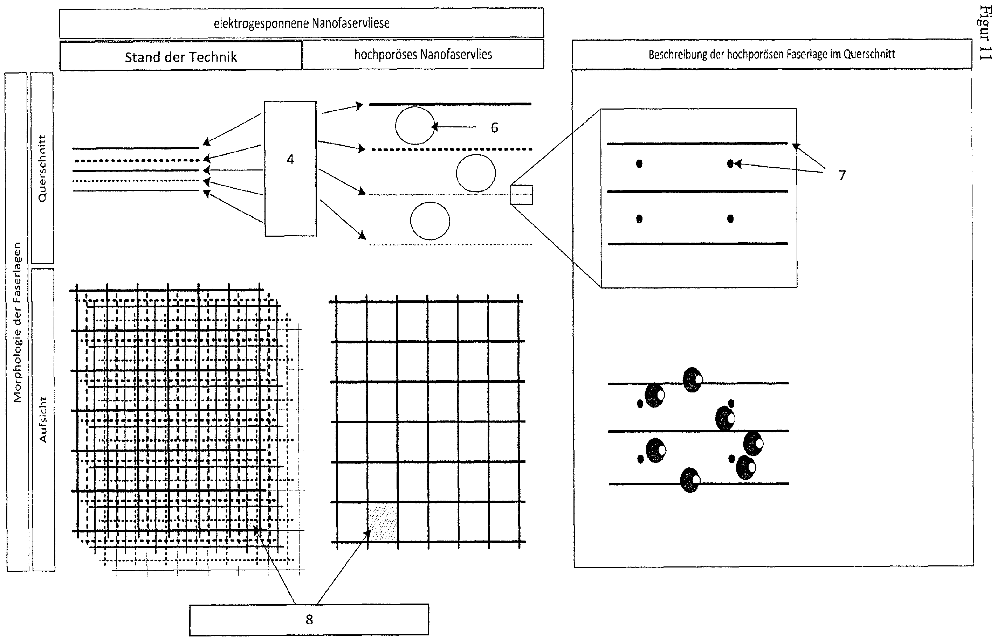

Figur 11    Schematische Zeichnung zur Erläuterung der Positionierung der Maschen in einem Nanofaservlies und des Aufbaus des Nanofaservlieses

Teil 1: linkes Bild "Elektronengesponnene Nanofaservliese"

Als Nanofaserlagen (4) werden in diesem Zusammenhang insbesondere dünne Fasergelege bezeichnet, welche bevorzugt im Herstellungsprozess durch Porogene (6) voneinander separiert werden. Nach Herauslösen der Porogene (6) bleiben die Nanofaserlagen (4) voneinander getrennt und bilden mit Flüssigkeit gefüllte Poren (5). Im dichten nicht-erfindungsgemäßen Nanofaservlies werden keine Porogene (6) eingearbeitet, weswegen keine Separierung der Nanofaserlagen (4) existiert und diese direkt übereinanderliegen. Durch die hohe Anzahl an Nanofasern (7) und Nanofaserlagen (4) im direkten Kontakt übereinander reduziert sich die Maschenweite (Betrachtung von oben auf die Fläche des dichten nicht-erfindungsgemäßen Nanofaservlieses). Die Separation der Nanofaserlagen (4) im hochporösen erfindungsgemäßen Nanofaservlies limitiert die Anzahl an Nanofasern (7) übereinander und garantiert damit die große Maschenweite (schraffierte Fläche, 8) des hochporösen erfindungsgemäßen Nanofaservlieses. Für die Erhaltung dieser hohen Maschenweite (und damit die Erhaltung der Zugänglichkeit durch Zellen) sind bevorzugt die einzelnen Nanofaserlagen ständig durch Flüssigkeit voneinander separiert zu halten, da durch attraktive Wechselwirkungen zwischen den Nanofasern (7) die Poren (5) verschwinden können und damit die Maschenweite stark abnimmt. Daher ist das hochporöse Nanofaservlies bevorzugt permanent in Flüssigkeit zu halten. Bei einem kurzzeitigeren Transfer außerhalb der Flüssigkeit ist bevorzugt zu gewährleisten, dass diese nicht komplett aus den Poren (5) fließt und damit die Nanofaserlagen (4) weiterhin separiert sind.

Teil 2: rechtes Bild "Beschreibung der hochporösen Nanofaserlage im Querschnitt"

Obere Darstellung: Die einzelnen Nanofaserlagen (4) des hochporösen erfindungsgemäßen Nanofaservlieses bestehen aus einer begrenzten Anzahl an freibeweglichen Nanofasern (7) übereinander, was insbesondere bewirkt, dass eine hochflexible Nanofaserlage (4) mit dynamischer Struktur erhalten wird. Die Anzahl der übereinanderliegenden Nanofasern (alle Orientierungen, 7) ist bevorzugt zwischen 1 und 25 Einzelfasern. Eine hohe Anzahl an Nanofasern (7) kann sich gegebenenfalls in einer Reduzierung der Maschenweite (schraffierte Fläche, 8) auswirken. Neben der Maschenweite bewirkt die bevorzugt geringe Nanofaseranzahl in der Nanofaserlage (4), dass die Berührungspunkte zwischen den Nanofasern minimiert werden und die Einzelnanofasern (7) auch über die Dicke der Nanofaserlage (4) zum Großteil voneinander getrennt vorliegen. Dies erlaubt es, dass die Einzelnanofasern (7) in der Nanofaserlage (4) in alle Raumrichtungen frei beweglich sind. Untere Darstellung: Werden nun Zellen (schwarze Punkte mit weißen kleineren Punkten) auf die Nanofaserlage (4) gegeben, können diese sich einfach zwischen den Nanofasern (7) bewegen und auch die einzelnen Nanofasern (7) verschieben. Dies Nanofaserlage (4) dient als lokal begrenztes 3D-Zellgerüst. Die Nanofaserlage (4) bleibt hochflexibel, sodass Zellverbände die Nanofaserlagen (4) zueinander ziehen können. Befinden sich zu viele Nanofasern (7) in der Nanofaserlage, reduziert sich die Beweglichkeit der Einzelnanofasern (7) erheblich und der 3D-Charakter der Nanofaserlage (4) geht für die Zellen verloren.

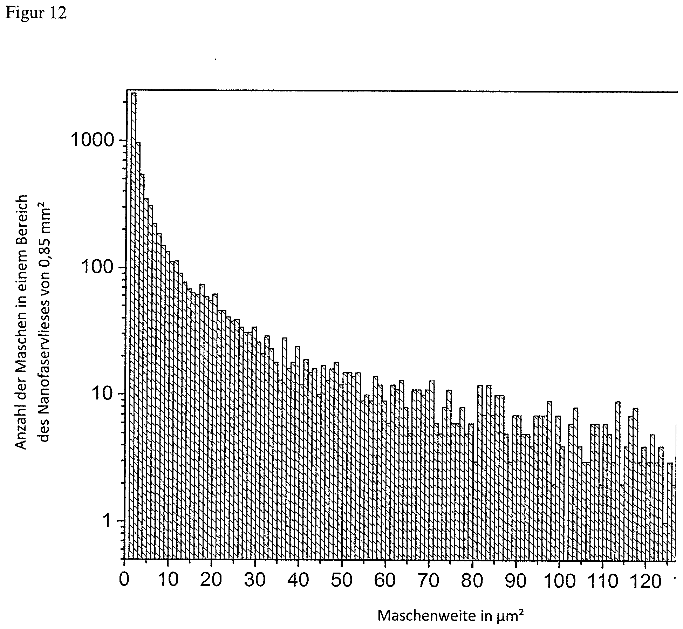

Figur 12    Darstellung der Anzahl an Maschen bestimmter Maschenweiten in einem erfindungsgemäßen Nanofaservlies aus PA6 (Polyamid) als Nanofasermaterial, hergestellt mit NaCl-Partikeln als Porogen und mit einer rotierenden Walze während des Elektroverspinnens entsprechend Beispiel 1 (Herstellungsbeispiel 1 "Aufbau rotierende Walze"). Auf der x-Achse ist die Fläche der Maschenweite in $\mu m^2$ angegeben und auf der y-Achse die Anzahl der Maschen, wobei die untersuchte Fläche des Nanofaservliesstückes 0,85 mm$^2$

betrug.

Beispiele

Beispiel 1

Herstellungsbeispiele/Kombination von Elektrospinnen mit additiven Fertigungen

Die Herstellung der hochporösen Nanofaservliese erfordert das Einbringen von Strukturen im Bereich von 50 - 500 $\mu$m zwischen die Nanofasern (7). Dieser Größenbereich kann mit verschiedenen Varianten von additiven Fertigungen abgedeckt und im automatisierten oder manuellen Modus gefahren werden. Neben diesen beiden Möglichkeiten können die kombinierten Verfahrensschritte von Verfahrensschritt b), nämlich des Elektroverspinnens und des Einbringens des Porogens (6), über einen alternierenden oder kontinuierlichen Prozess gestaltet werden. Bevorzugt bei allen Methoden ist, dass diese Mikro-Strukturen während des Spinnprozesses auf/zwischen den Nanofasern halten können, insbesondere durch die Fliehkräfte bei rotierender Walze.

Herstellungsbeispiel 1: Partikel

Diese Herstellungsvariante verwendet Partikel als Porogen (6) zwischen den Nanofasern (7). Die Partikel können dabei händisch mittels eines Streuers oder über einer automatisch geregelten Streuvorrichtung auf das Nanofaservlies dosiert werden. Im Folgenden sind zwei Varianten des Prozesses beschrieben.
Materialbeispiele für diese partikularen Porogene sind: NaCl, NaCO$_3$, PEG/PEO, Zucker.

Aufbau rotierende Walze:

In diesem Aufbau kann ein alternierender Prozess gefahren werden. Dies bedeutet, dass der Spinnprozess nach einer bestimmten Zeit angehalten, die Partikel (6) aufgebracht und anschließend weitergesponnen wird (Figur 3A). Durch die Rotation der Walze muss zusätzlich die Haftkraft der Partikel (6) auf den Nanofasern (7) erhöht werden. Bei wasserlöslichen Partikeln (zum Beispiel NaCl, 6) wird die Nanofaseroberfläche zunächst mit Ethanol befeuchtet und anschließend mit Partikeln (6) bestreut. Die geringe Löslichkeit des Porogenmaterials löst die Partikel (6) oberflächlich an und klebt diese dadurch an die Nanofasern (7). In diesem Prozess kann von zwei Seiten mit jeweils mindestens einer Spinn-Quelle gesponnen werden.
Beispielsweise konnte mit dieser Ausführungsform ein erfindungsgemäßes Nanofaservlies hergestellt werden, welches PA6 (Polyamid) als Nanofasermaterial und NaCl-Partikel als Porogen umfasst. Die Porosität eines solchen Nanofaservlieses betrug 98,2 % (gemessen mittels konfokalem Reflexionsmikroskop).
Die NaCl-Partikel hatten einen Durchmesser von 30 bis 80 $\mu$m.
Der Durchmesser der Nanofasern betrug 371 $\pm$ 192 nm.

Aufbau Platte/planare Oberfläche

Dieser Aufbau benötigt keine zusätzliche Erhöhung der Porogenhaftung und kann daher sowohl über einen kontinuierlichen als auch einen alternierenden Prozess gefahren werden. Hierbei befindet sich mittig über dem Target ein Streuer, welcher gleichmäßig die Fläche mit den Partikeln (6) bestäubt (Figur 3B). Etwas seitlich befinden sich die Spinn-Quellen, welche ebenfalls möglichst gleichmäßig die Nanofasern (7) auf dem Target verteilen. Je nach Größe der Platte können dabei auch eine Vielzahl an Streu- und Spinnquellen eingesetzt werden. Im kontinuierlichen Prozess ist zu beachten, dass das partikuläre Porogen (6) nicht mit dem elektrischen Feld der Spinnquellen interagiert und damit die gleichmäßige Streuung negativ beeinflusst.
Figur 3 C und D zeigt resultierende Nanofaservliese mit NaCl-Partikeln (6) zwischen den Nanofasern (7) und Figur 1 das resultierende hochporöse Nanofaservlies mit zufällig orientierten (A, B) und gerichteten Nanofasern (C, D).

Herstellungsbeispiel 2: Kombination mit Mikrofasern beispielsweise über Druckspinnverfahren

Diese Variante verwendet Fasern als Porogen (6) zwischen den Nanofasern (7). Dieser Prozess kann sowohl alternierend als auch kontinuierlich durchgeführt werden. Im kontinuierlichen Prozess müssen Wechselwirkungen zwischen Mikrofaser (6) und elektrischem Feld ausgeschlossen sein, da sonst das Ablegen der Fasern (6) zu ungenau beziehungsweise auch unmöglich ist. Materialbeispiele für das Druckspinnen sind: Hybridmaterialien auf Sol-Gel-basis, Ethanol-lösliche Polymere (z.B. PVA).

Alternierendes Spinnen auf eine Platte:

Eine Ausführungsform stellt ein alternierender Prozess durch Spinnen auf einer Platte (Platte wechselt in definierten Zeitabständen die Spinnanlagen) dar (Abb. 4A). In sehr kurzen Zeitabständen (20 - 60 s) erfolgt ein abwechselndes Spinnen von Mikro- (6) und Nanofasern (7). Es resultiert ein schichtartiger Aufbau des hochporösen Nanofaservlieses in Form von Nanofaserlagen (4) mit nur sehr geringem Zusammenhalt zwischen den einzelnen Nanofaserlagen (4). Figur 5A, B zeigt beispielhaft ein Nanofaservlies durch die Kombination des Druckspinnens mit dem Elektrospinnen. Im Vergleich zur Partikelmethode lassen sich hier weit dickere hochporöse Nanofaservliese erzeugen (Figur 5C).

Gemäß dieser Ausführungsform wurde ein Nanofaservlies hergestellt, welches PA6 (Polyamid) als Nanofasermaterial und Titan-Oxo-Carboxo-Cluster Mikrofasern als Porogen umfasst. Die Porosität eines solchen Nanofaservlieses betrug 99,1 % (gemessen mittels konfokalem Reflexionsmikroskop).

Aufbau rotierende Walze:

In diesem Aufbau können der Elektrospinnprozess und der Druckspinnvorgang kontinuierlich gefahren werden (nicht zwingend; alternierend ist ebenfalls möglich). Während von den Seiten die Nanofaserspinnquellen lokalisiert sind, erfolgt der Druckspinnvorgang von oben direkt auf die Walze (Figur 4B). Für eine bessere Verteilung der Mikrofasern (6) kann der Druckspinnkopf in Ausdehnungsrichtung der Walze kontinuierlich hin und her bewegt werden. Erzeugen die Nanofaserquellen auf beiden Seiten ein ähnliches elektrisches Feld, ist es auch möglich, den Einfluss des Feldes auf die fallenden Mikrofasern (6) auszugleichen.

Ausrichtung Nanofaserquellen senkrecht zur Fallrichtung der Mikrofasern (6):

In diesem Aufbau wird die Spinn- / Flugrichtung der Nanofasern (7) senkrecht zur Fallrichtung der Mikrofasern (6) orientiert, indem auf einer Seite des Fallweges die Nanofaserquellen und auf der anderen Seite die geerdete Quelle platziert werden (Figur 4C). Die Nanofasern (7) werden dabei im Flug von den Mikrofasern (6) aufgefangen und auf den unteren Kollektor gesammelt. Vorteilhaft in dieser Methode ist, dass die Dicke des erzeugten Nanofaservlieses nicht den Spinnvorgang beeinflusst und das Nanofaservlies damit wesentlich dicker generiert werden kann.

Aufbau Elektrospinnen kombiniert mit dem Abwickeln eines bereits vorhandenen Mikrofadens:

Sollte die Porogen-Faser (6) schon im Vorfeld erzeugt sein, kann in dieser Variante die Faser (6) während des Spinnprozesses kontinuierlich von einer Spule abgewickelt und an der rotierenden Walze aufgewickelt werden (Figur 4D). Dabei bewegt sich die Spule kontinuierlich parallel zur Walze hin und her und kann damit die Ablage des Fadens steuern. Figur 5D zeigt beispielhaft die Kombination dieser Mikrofasern (6) mit den elektrogesponnenen Nanofasern (7). Im Vergleich zur Partikelmethode lassen sich hier weit dickere hochporöse Nanofaservliese erzeugen (Figur 5E).

Herstellungsbeispiel 3: Kombination mit 3D-Druck; beispielsweise Fused Deposition Modeling

(FDM), Bioprinting/Bioplotting oder Melt Electro Writing (MEW). Diese Verfahren erlauben die genaue Positionierung des Porogens (6) im Nanofaservlies. Über FDM können dabei sowohl partikuläre als auch faserförmige Porogene (6) erzeugt werden. Am geeignetsten ist hier der alternierende Prozess, in welchem zwischen Spinnen und Drucken abgewechselt wird. Dies kann entweder als ein kombinierter Prozess auf einer rotierenden Walze (Figur 6A) stattfinden oder getrennt auf einer Platte, welche zwischen den Prozessen wechselt (Figur 6B). Als Material eignen sich wasser- oder ethanollösliche Thermoplasten, wie beispielsweise PVA. Das Verfahren des MEW beschreibt ein ähnliches Verfahren, welches zusätzlich ein elektrisches Feld nutzt, um die Fasern möglichst genau abzulegen. Neben FDM kann ebenfalls das Verfahren des Bioprintings/Bioplottings angewendet werden, welches statt geschmolzenem Kunststoff Biotinten aus Hydrogelen verwendet.

Beispiel 2 Bestimmung der Porositäten und Volumenverhältnisse von Nanofaservliesen

Beispiel 2a Bestimmung der Porosität

- Ausschneiden eines Stück Nanofaservlies (mit Porogen (6)) und Bestimmung der makroskopischen Oberfläche (A): A = 380 mm$^2$

- Herauslösen des Porogens (6) in Wasser mit anschließender Trocknung der Fasern und unmittelbar erfolgender Bestimmung der Trocken-Fasermasse: $m_{Faser}$ = 1,081 mg
- Ausschneiden eines weiteren Stückes (mit Porogen (6)). Anschließend Herauslösen des Porogens (6) mit Wasser und unmittelbar erfolgende Einbettung des hochporösen Nanofaservlieses (Cryo-, Paraffin- oder sonstige Kunststoffeinbettung). Bedeutsam ist, dass ein Austausch des Wassers mit dem flüssigen Einbettmittel erfolgt, bevor dieser aushärtet. Im Falle der Polyamid-Fasern (7) wurde das Vlies über eine aufsteigende Alkoholreihe in Paraffin eingebettet.
- Erzeugung von Dünnschnitten (Im Falle der Paraffineinbettung mit einem Standart-Mikrotom) und Transfer des Schnittes auf einen Objektträger
- Entfernung des Einbettmittels mit anschließender Einbettung in Mikroskopie-geeignetes Einbettmittel inklusive Deckglas
- Bestimmung der Dicke des hochporösen Nanofaservlieses (12) über Mikroskopie: d = 200 $\mu$m
- Bestimmung des Gesamtvolumens: $V_{Gesamt}$ = d $\times$ A = 200 $\mu$m $\times$ 380 mm$^2$ = 76 mm$^3$
- Bestimmung des Faservolumens

$$V_{Fasern} = m_{Faser} / Dichte_{Polyamid6} = 1{,}081\ mg / 1{,}084\ g/cm^3 = 0{,}997\ mm^3$$

- Bestimmung der Porosität:

Porosität P = ($V_{Gesamt}$ - $V_{Fasern}$) / $V_{Gesamt}$ $\times$ 100 = (76 - 0,997) mm$^3$ / 76 mm$^3$ $\times$ 100 = <u>98,69 %</u>        (Formel (A)):

Porosität P = ($Dicke_{poröses\ Faservlies}$ $\times$ $A_{makroskopisch}$ - $m_{Faser}$/$Dichte_{Fasermaterial(Polyamid6)}$)/( $Dicke_{poröses\ Faservlies}$ $\times$ $A_{makroskopisch}$) $\times$ 100        (Formel (B)):

<u>Beispiel 2b Bestimmung der Porosität</u>

1. Vorbereitung:

- Porogene aus dem Faservlies lösen (beispielsweise Wasser)
- Faservlies in einem Gefäß mit Deckglasboden positionieren
- Faservlies mit Metallring fixieren (Innendurchmesser des Metallrings: 1 cm), wobei keine mechanische Belastung auf das Nanofaservlies ausgeübt wurde.
- Gefäß mit Wasser auffüllen und im konfokalen Mikroskop (beispielsweise Leica LSM SP8) positionieren

2. Messparameter:

- Laser: 476 nm; Argon - Laser; Grundleistung 25%; davon Leistung auf Probe zwischen 30 und 70%
- Detektor: 476 nm $\pm$ 5 nm
- Detektorverstärkung: 50 - 100
- Objektiv: 40$\times$
- Bildfläche (x-y): 1024 $\times$ 1024 Pixel / 290,91 $\times$ 290,91 $\mu$m
- Bildtiefe (z): 350 - 450 $\mu$m
- Gradient der Laser-Leistung und der Detektorverstärkung über die Tiefe/Dicke der Probe

3. Auswertung:

- Software ImageJ
- Anpassung von Helligkeit und Kontrast im kompletten Z-Stack
- Binärisierung des kompletten Z-Stack (nur schwarz-weiß)
- Auszählung der schwarzen und weißen Voxel und Berechnung des Volumenanteils (= Porosität)

<u>Beispiel 2c Bestimmung der Volumenverhältnisse</u>

Zur Bestimmung des Volumenverhältnisses von Porogenmaterial zu Nanofasermaterial wird wie folgt vorgegangen:

1. Trocknung des Nanofaservlieses nach dem Spinnprozess
2. Bestimmung der Gesamtmasse (Porogen und Nanofasern)
3. Herauslösen des Porogens
4. Trocknen des Nanofaservlieses
5. Bestimmung der Masse des Nanofaservlieses
6. Berechnung der Masse des Porogens aus Gesamtmasse und Masse des Nanofaservlieses
7. Berechnung der Volumina von Porogenmaterial und Nanofasermaterial und Berechnung von deren Verhältnis

Beispiel 3

Anwendungsbeispiel Matrix Stromale Gewebe

Das Bindegewebe stellt eine der wichtigsten Bestandteile von Organen oder Organmodellen dar. Das hier offenbarte Nanofaservlies eignet sich für eine Vielzahl an Geweben wie: Herz, Atemwege, Darm, Knochen, Knorpel, Niere, Urogenitaltrakt, Leber oder Gefäße. Dabei können gewebespezifische Eigenschaften über die Verwendung von Fibroblasten aus dem jeweiligen Gewebe erzeugt werden und die stromale Matrix dann mit dem jeweiligen epithelialen Gewebe verbunden werden. Beispielhaft wird der Aufbau eines Hautmodells beschrieben, bei welchem die Dermis auf dem hochporösen Nanofaservlies basiert.

In diesem Beispiel werden Nanofaservliese verwendet, welche gemäß dem Herstellungsbeispiel 1 "Aufbau rotierende Walze" (siehe Beispiel 1) hergestellt wurden. Die Maschengröße und -anzahl kann der Figur 12 entnommen werden.

Zunächst erfolgt das Zuschneiden des trockenen Nanofaservlieses (mit Porogen (6)) auf die gewünschte Größe (z.B. $2{,}5 \times 2{,}5$ cm). Anschließend wird das Porogen (6) im geeigneten Lösungsmittel (z.B. Wasser) herausgelöst und das Nanofaservlies in der Kulturvorrichtung eingespannt. Dazu kann beispielsweise eine Zellkrone oder ein Transwell®-Insert verwendet werden. Je nach gewünschter Dicke des dermalen Parts können beispielsweise 2 oder 4 Nanofaservliese mit Dicken von 200 $\mu$m übereinandergestapelt werden (bei dickeren Nanofaservliesen entsprechend weniger). Anschließend erfolgt die Aussaat von humanen Fibroblasten (40000 Zellen/cm$^2$) auf dem Nanofaservlies. Nach 2 - 4 Wochen Kultur erfolgt die komplette Besiedlung des Nanofaservlieses sowie die Synthese von Matrixproteinen innerhalb der Poren (5) und Maschen (8). Nach dieser Biologisierung des Nanofaservlieses werden humane Keratinozyten (600000 Zellen/cm$^2$) ausgesät und für 3 Wochen unter Airlift-Bedingungen kultiviert. In dieser Zeit bildet sich die Epidermis auf dem Nanofaserbasiertem Bindegewebe aus und resultiert in einem ca. 1 cm$^2$ großem Hautmodel auf Basis synthetischer Nanofasern (Figur 7A). Die Anwendung des biologisierten Nanofaservlieses führt zu einer vollausgebildeten Epidermis mit allen relevanten Schichten und einer Dicke von 150 - 200 $\mu$m (Figur 7B). Eine IF-Färbung (Immunofluoreszenz-Färbung) gegen Vimentin zeigt eine homogene Verteilung der Fibroblasten im stromalen Gewebe des Faservlieses (Figur 7C). Des Weiteren sieht man in der Nahaufnahme, dass sich die Zellen hauptsächlich an den Nanofasern (7) befinden. Betrachtet man dagegen die Collagen I Verteilung, befindet sich dieses Matrixprotein sowohl an den Nanofasern (7) als auch in den Poren (5) (Figur 7D). IF-Färbungen gegen differenzierungsspezifische Marker der Epidermis bestätigen deren ausgereiften Status (Figur 7E) sowie die Ausbildung einer Basalmembran (Figur 7F).

Anwendungsbeispiel Verbindung von stromalen mit hochporösen Geweben wie Fettgewebe

Oft bestehen Organe nicht nur aus einem Gewebetyp, sondern stromale Gewebe wechseln sich mit hochporösen Geweben ab und stehen in kontinuierlichen Austausch miteinander. Das hier beschriebene Nanofaservlies ermöglicht es, beide Typen zu verbinden, was im Folgenden an der Verbindung von der Lederhaut (Dermis) mit der Unterhaut (Subcutis) gezeigt wird.

Die Dermis beschreibt in der Haut nur den obersten Bindegewebeteil dieses Organs. Die nächsttiefere Schicht ist die Subcutis, welche hauptsächlich aus Fettgewebe aufgebaut ist. Durch die adipogene Differenzierung von mesenchymalen Stromazellen (MSCs) kann der zelluläre Anteil von Fettgewebe erzeugt werden. Durch die starke Volumenzunahme sind als Scaffold nur sehr poröse Strukturen geeignet. Des Weiteren können MSCs nicht von Anfang an mit Keratinozyten in einem Modell kombiniert werden, da die verschiedenen Medien die Differenzierung der verschiedenen Zellen gegenseitig negativ beeinflussen. Der Vorteil dieser hochporösen Nanofaservliese ist, dass beide Zelltypen getrennt voneinander das Nanofaservlies besiedeln und anschließend getrennt differenzieren.

Abschließend können die Nanofaservliese zusammengesetzt werden und das Gewebe kann über einen bestimmten Zeitrahmen reifen.

Im Detail erfolgt die Herstellung des oberen Teils (Dermis - Epidermis analog zum vorigen Textabschnitt). Für die Generierung des Fettgewebes werden MSCs (100000 Zellen/cm$^2$) auf dem Faservlies ausgesät und zunächst für 2 Wochen kultiviert. Anschließend erfolgt die adipogene Differenzierung für weitere 2 Wochen. Nach dieser Zeit ist diese Differenzierung so weit fortgeschritten, dass ein Wechsel vom adipogenen Differenzierungsmedium zu Keratinozytenmedium keinen negativen Einfluss auf diese Zellen hat. Daher kann nun das adipogene Gewebe von unten an das Hautmodell angebracht werden und gemeinsam für 2-3 Wochen reifen. Figur 8A, B zeigt das resultierende 3-schichtige Hautmodel auf Basis der hochporösen Nanofaservliese. Die Fetttröpfchen befinden sich in der HE-Färbung (Figur 8A) an den Positionen der Löcher, da diese durch da das Fett durch das Einbettungsverfahren (Paraffin) herausgewaschen wurde. Durch die Anwendung der Cryoeinbettung bleiben die Lipide erhalten und können eingefärbt werden. Beispielsweise über den Fluoreszenzfarbstoff Nile Red (Figur 8B) oder dem roten Farbstoff Ölrot (Figur 8C). Die Färbung mit Ölrot kann zudem die Nanometer-skaligen Fetttröpfchen in der obersten Schicht der Epidermis sichtbar machen (Figur 8D).

## Patentansprüche

1. Nanofaservlies umfassend ein Netzwerk von aus mindestens einem Nanofasermaterial aufgebauten, Poren (5) umschließenden Nanofasern (7), hergestellt durch ein Verfahren umfassend die Verfahrensschritte:

    a) Bereitstellen mindestens eines Nanofasermaterials und mindestens eines zur Ausbildung mindestens eines Porogens geeigneten Porogenmaterials,
    b) Elektroverspinnen des mindestens einen Nanofasermaterials unter Einbringen von aus dem mindestens einem Porogenmaterial ausgebildeten mindestens einem Porogen (6) **dadurch gekennzeichnet, dass** das Porogen (6) einen Durchmesser von 30 bis 1000 $\mu$m aufweist, so dass das Volumenverhältnis von Porogenmaterial zu Nanofasermaterial in dem nach Abschluss dieses Verfahrensschrittes erhaltenen elektroversponnenen Nanofaservlies 40 bis 99 zu 60 bis 1 (jeweils Volumen, bezogen auf das Gesamtvolumen, also Fasern und Porogene, des Nanofaservlieses) beträgt und
    c) Erhalten eines Nanofaservlieses umfassend mindestens ein Porogen (6),
    wobei die Nanofasern in Form von Nanofaserlagen vorliegen, wobei eine Pore ein von Nanofaserlagen umschlossener Raum ist, welcher durch Porogene, Zellen oder Fluide füllbar ist, wobei die Poren die einzelnen Nanofaserlagen voneinander räumlich trennen,
    wobei eine Nanofaserlage eine Schicht aus über- und/oder nebeneinander angeordneten Nanofasern ist, und wobei sofern das Porogen in Verfahrensschritt a) als fertig ausgebildetes Porogen bereitgestellt wird, in Verfahrensschritt b) alternierend ein Einbringen des Porogens und das Elektroverspinnen des Nanofasermaterials durchgeführt wird, oder
    sofern das in Verfahrensschritt a) bereitgestellte Porogenmaterial ein zum Ausbilden mindestens eines faserförmigen Porogens geeignetes Material ist, in Verfahrensschritt b) aus dem Porogenmaterial vor oder während dessen Einbringen in das elektroversponnene Nanofasermaterial mindestens ein faserförmiges Porogen ausgebildet wird.

2. Nanofaservlies gemäß Anspruch 1, wobei das Verfahren ferner die folgenden Verfahrensschritte umfasst:

    d) Inkubieren des Nanofaservlieses erhalten in Verfahrensschritt c) umfassend mindestens ein Porogen (6) in mindestens einem Lösungsmittel zum mindestens teilweisen Entfernen des mindestens einen Porogens aus dem Nanofaservlies und
    e) Erhalten eines Nanofaservlieses.

3. Nanofaservlies gemäß Anspruch 1 oder 2, wobei das in Verfahrensschritt a) bereitgestellte Porogenmaterial partikelförmig oder faserförmig ist, einen Durchmesser von 30 bis 1000 $\mu$m aufweist und in Verfahrensschritt a) als ausgebildetes Porogen (6) bereitgestellt wird.

4. Nanofaservlies gemäß Anspruch 1 oder 2, wobei das in Verfahrensschritt a) bereitgestellte Porogenmaterial ein zum Ausbilden mindestens eines faserförmigen Porogens (6) geeignetes Material ist und in Verfahrensschritt b) aus dem Porogenmaterial vor oder während dessen Einbringen in das elektroversponnene Nanofasermaterial mindestens ein faserförmiges Porogen ausgebildet wird.

5. Nanofaservlies umfassend ein Netzwerk von aus mindestens einem Nanofasermaterial aufgebauten, Poren (5) umschließenden Nanofasern (7), wobei die Porosität des Nanofaservlieses 90,0 bis 99,9 Vol.-%, insbesondere 98,5 bis 99,9 Vol.-% (jeweils bezogen auf Gesamtvolumen des Nanofaservlieses) beträgt.

6. Nanofaservlies nach Anspruch 1 bis 5, wobei in dem Nanofaservlies mindestens eine Flüssigkeit vorliegt, insbesondere 90,0 bis 99,9 Vol.-% Flüssigkeit (bezogen auf Gesamtvolumen des Nanofaservlieses).

7. Nanofaservlies nach einem der Ansprüche 1 oder 3 bis 6, wobei das Nanofaservlies zusätzlich Porogene (6) umfasst.

8. Nanofaservlies nach Anspruch 7, wobei die Porogene (6) partikelförmig oder faserförmig sind und/oder einen Durchmesser von 30 bis 1000 $\mu$m aufweisen.

9. Verfahren zur Herstellung eines Nanofaservlieses umfassend ein Netzwerk von aus mindestens einem Nanofasermaterial aufgebauten, Poren (5) umschließenden Nanofasern (7) nach Ansprüchen 1 bis 8, umfassend folgende Verfahrensschritte:

   a) Bereitstellen mindestens eines Nanofasermaterials und mindestens eines zur Ausbildung mindestens eines Porogens geeigneten Porogenmaterials,
   b) Elektroverspinnen des mindestens einen Nanofasermaterials unter Einbringen von aus dem mindestens einem Porogenmaterial ausgebildeten mindestens einem Porogen (6) **dadurch gekennzeichnet, dass** das Porogen (6) einen Durchmesser von 30 bis 1000 $\mu$m aufweist, so dass das Volumenverhältnis von Porogenmaterial zu Nanofasermaterial in dem nach Abschluss dieses Verfahrensschrittes erhaltenen elektroversponnenen Nanofaservlies 40 bis 99 zu 60 bis 1 (jeweils Volumen, bezogen auf das Gesamtvolumen, also Fasern und Porogene, des Nanofaservlieses) beträgt und
   c) Erhalten eines Nanofaservlieses umfassend mindestens ein Porogen (6), insbesondere mit einer Porosität von 90,0 bis 99,9 Vol.-%, insbesondere 98,5 bis 99,9 Vol.- % (jeweils bezogen auf Gesamtvolumen des Nanofaservlieses), bevorzugt mit einer Porosität von 90,0 bis 99,9 Vol.-%, insbesondere 98,5 bis 99,9 Vol.- % (jeweils bezogen auf Gesamtvolumen des Nanofaservlieses) und Maschen (8) mit einer Weite von 10 bis 200 $\mu$m$^2$ (gemessen in Wasser ohne Porogen (6)),
   wobei die Nanofasern in Form von Nanofaserlagen vorliegen, wobei eine Pore ein von Nanofaserlagen umschlossener Raum ist, welcher durch Porogene, Zellen oder Fluide füllbar ist, wobei die Poren die einzelnen Nanofaserlagen voneinander räumlich trennen,
   wobei eine Nanofaserlage eine Schicht aus über- und/oder nebeneinander angeordneten Nanofasern ist, und wobei sofern das Porogen in Verfahrensschritt a) als fertig ausgebildetes Porogen bereitgestellt wird, in Verfahrensschritt b) alternierend ein Einbringen des Porogens und das Elektroverspinnen des Nanofasermaterials durchgeführt wird, oder
   sofern das in Verfahrensschritt a) bereitgestellte Porogenmaterial ein zum Ausbilden mindestens eines faserförmigen Porogens geeignetes Material ist, in Verfahrensschritt b) aus dem Porogenmaterial vor oder während dessen Einbringen in das elektroversponnene Nanofasermaterial mindestens ein faserförmiges Porogen ausgebildet wird.

10. Verfahren zur Herstellung eines Nanofaservlieses nach Anspruch 9, ferner umfassend die Verfahrensschritte:

    d) Inkubieren des Nanofaservlieses erhalten in Verfahrenschritt c) umfassend mindestens ein Porogen (6) in mindestens einem Lösungsmittel zum mindestens teilweisen Entfernen des mindestens einen Porogens aus dem Nanofaservlies und
    e) Erhalten eines Nanofaservlieses.

11. Verfahren zur Herstellung eines Nanofaservlieses nach einem der Ansprüche 9 oder 10, wobei das in Verfahrensschritt a) bereitgestellte Porogenmaterial partikelförmig oder faserförmig ist, einen Durchmesser von 30 bis 1000 $\mu$m aufweist und in Verfahrensschritt a) als ausgebildetes Porogen (6) bereitgestellt wird, oder
    wobei das in Verfahrensschritt a) bereitgestellte Porogenmaterial ein zum Ausbilden mindestens eines faserförmigen Porogens (6) geeignetes Material ist und in Verfahrensschritt b) aus dem Porogenmaterial vor oder während dessen Einbringen in das elektroversponnene Nanofasermaterial mindestens ein faserförmiges Porogen ausgebildet wird.

12. Verwendung eines Nanofaservlieses nach einem der Ansprüche 1 bis 8 oder hergestellt nach einem Verfahren nach einem der Ansprüche 9 bis 11 zur Kultivierung oder Differenzierung von Zellen oder zur nicht-therapeutischen Behandlung von Menschen oder Tieren.

13. Künstliches Gewebe umfassend ein Nanofaservlies nach einem der Ansprüche 1 bis 8 oder herstellbar nach dem Verfahren nach einem der Ansprüche 9 bis 11 und mindestens eine Zelle mindestens eines Zelltyps.

14. Künstliches Gewebe nach Anspruch 13, wobei das künstliche Gewebe 1 bis 50 Gew.-% Nanofaservlies und 50 bis 99 Gew.-% Zellen, insbesondere 80 bis 99 Gew.-%, und, optional, extrazelluläre Matrix umfasst (jeweils bezogen auf das Gesamtgewicht des künstlichen Gewebes) und/oder

wobei das im Nanofaservlies vorliegende mindestens eine Porogen (6) vor der Besiedelung mit der mindestens einer Zelle mindestens eines Zelltyps aus dem Nanofaservlies mindestens teilweise entfernt wurde.

15. Verfahren zur Herstellung eines künstlichen Gewebes nach einem der Ansprüche 13 bis 14 umfassend folgende Verfahrensschritte:

x1) Bereitstellen einer Zelle mindestens eines Zelltyps, einem Kulturmedium und mindestens eines Nanofaservlieses nach einem der Ansprüche 1 bis 8 oder herstellbar nach einem Verfahren nach einem der Ansprüche 9 bis 11,

x2) optionales Entfernen mindestens eines Teils eines gegebenenfalls vorhandenen mindestens eines Porogens (6),

x3) Kultivieren des mindestens einen Nanofaservlieses mit der mindestens einen Zelle des mindestens einen Zelltyps in dem Kulturmedium und

x4) Erhalten eines künstlichen Gewebes umfassend mindestens eine auf oder in dem mindestens einen Nanofaservlies angeordnete Zelle.

16. Verfahren nach Anspruch 15, wobei während oder nach der Kultivierung gemäß Verfahrensschritt x3) das Vlies in einem Verfahrensschritt x31) mit mindestens einer Zelle mindestens eines weiteren Zelltyps, insbesondere zu dem in Verfahrensschritt x1) bereitgestellten Zelltyp unterschiedlichen Zelltyps, kultiviert wird und/oder

wobei während oder nach der Kultivierung gemäß Verfahrensschritt x3) oder x31) weitere Nanofaservliese, bevorzugt besiedelt mit mindestens einer Zelle eines gleichen oder anderen Zelltyps, auf das Nanofaservlies, erhalten in x3) oder x31), gestapelt werden.

17. Nanofaservlies nach einem der Ansprüche 1 bis 8 oder hergestellt nach einem Verfahren nach einem der Ansprüche 9 bis 11 zur Verwendung in der Behandlung von erkrankten Menschen oder Tieren, insbesondere zur Implantation.

**Claims**

1. Nanofibre nonwoven comprising a network of nanofibres (7) composed of at least one nanofibre material and enclosing pores (5), produced by a method comprising the method steps:

a) providing at least one nanofibre material and at least one porogen material suitable for formation of at least one porogen,,

b) electrospinning the at least one nanofibre material while introducing at least one porogen (6) formed from the at least one porogen material, **characterised in that** the porogen (6) has a diameter of 30 to 1000 $\mu$m, so that the volume ratio of porogen material to nanofibre material in the electrospun nanofibre nonwoven obtained after completion of this method step is 40 to 99 to 60 to 1 (in each case volume based on the total volume, i.e. fibres and porogens, of the nanofibre nonwoven) and

c) obtaining a nanofibre nonwoven comprising at least one porogen (6),

wherein the nanofibres are present in the form of nanofibre layers, wherein a pore is a space enclosed by nanofibre layers, which can be filled by porogens, cells or fluids, wherein the pores spatially separate the individual nanofibre layers from each other,

wherein a nanofibre layer is a layer of nanofibres arranged above and/or next to one another, and

wherein, insofar as the porogen is provided in method step a) as a ready-formed porogen, in method step b) an introducing of the porogen and the electrospinning of the nanofibre material is carried out alternately, or

insofar as the porogen material is provided in method step a) as a material suitable for forming at least one fibrous porogen, in method step b) at least one fibrous porogen is formed from the porogen material before or during its introduction into the electrospun nanofibre material.

2. The nanofibre nonwoven according to claim 1, wherein the method further comprises the following method steps:

d) incubating the nanofibre nonwoven obtained in method step c) comprising at least one porogen (6) in at least one solvent to at least partially remove the at least one porogen from the nanofibre nonwoven, and

e) obtaining a nanofibre nonwoven.

3. The nanofibre nonwoven according to claim 1 or 2, wherein the porogen material provided in method step a) is particulate or fibrous, has a diameter of 30 to 1000 $\mu$m and is provided in method step a) as a formed porogen (6).

4. The nanofibre nonwoven according to claim 1 or 2, wherein the porogen material provided in method step a) is a material suitable for forming at least one fibrous porogen (6) and in method step b) at least one fibrous porogen is formed from the porogen material before or during its introduction into the electrospun nanofibre material.

5. The nanofibre nonwoven comprising a network of nanofibres (7) composed of at least one nanofibre material and enclosing pores (5), wherein the porosity of the nanofibre nonwoven is 90.0 to 99.9 vol.%, in particular 98.5 to 99.9 vol.% (in each case based on the total volume of the nanofibre nonwoven).

6. The nanofibre nonwoven according to claims 1 to 5, wherein at least one liquid is present in the nanofibre nonwoven, in particular 90.0 to 99.9 vol.% of liquid (based on the total volume of the nanofibre nonwoven).

7. The nanofibre nonwoven according to one of claims 1 or 3 to 6, wherein the nanofibre nonwoven additionally comprises porogens (6).

8. The nanofibre nonwoven according to claim 7, wherein the porogens (6) are particulate or fibrous and/or have a diameter of 30 to 1000 $\mu$m.

9. A method for producing a nanofibre nonwoven comprising a network of nanofibres (7) composed of at least one nanofibre material and enclosing pores (5) according to claims 1 to 8, comprising the following method steps:

a) providing at least one nanofibre material and at least one porogen material suitable for formation of at least one porogen,
b) electrospinning the at least one nanofibre material while introducing at least one porogen (6) formed from the at least one porogen material, **characterised in that** the porogen (6) has a diameter of 30 to 1000 $\mu$m, so that the volume ratio of porogen material to nanofibre material in the electrospun nanofibre nonwoven obtained after completion of this method step is 40 to 99 to 60 to 1 (in each case volume, based on the total volume, i.e. fibres and porogens, of the nanofibre nonwoven), and
c) obtaining a nanofibre nonwoven comprising at least one porogen (6), in particular with a porosity of 90.0 to 99.9 vol.%, in particular 98.5 to 99.9 vol.% (in each case based on the total volume of the nanofibre nonwoven), preferably having a porosity of 90.0 to 99.9 vol.%, in particular 98.5 to 99.9 vol.% (in each case based on the total volume of the nanofibre nonwoven) and meshes (8) with a width of 10 to 200 $\mu$m$^2$ (measured in water without porogens (6)),
wherein the nanofibres are present in the form of nanofibre layers, wherein a pore is a space enclosed by nanofibre layers, which can be filled by porogens, cells or fluids, wherein the pores spatially separate the individual nanofibre layers from each other,
wherein a nanofibre layer is a layer of nanofibres arranged above and/or next to one another, and
wherein, insofar as the porogen is provided in method step a) as a ready-formed porogen, in method step b) an introducing of the porogens and the electrospinning of the nanofibre material is carried out alternately, or insofar as the porogen material is provided in method step a) as a material suitable for forming at least one fibrous porogen, in method step b) at least one fibrous porogen is formed from the porogen material before or during its introduction into the electrospun nanofibre material.

10. The method for producing a nanofibre nonwoven according to claim 9, further comprising the method steps:

d) incubating the nanofibre nonwoven obtained in method step c) comprising at least one porogen (6) in at least one solvent to at least partially remove the at least one porogen from the nanofibre nonwoven, and
e) obtaining a nanofibre nonwoven.

11. The method for producing a nanofibre nonwoven according to claim 9 or 10, wherein the porogen material provided in method step a) is particulate or fibrous, has a diameter of 30 to 1000 $\mu$m and is provided in method step a) as formed porogen (6), or wherein the porogen material provided in method step a) is a material suitable for forming at least one fibrous porogen (6), and in method step b) at least one fibrous porogen is formed from the porogen material before or during its introduction into the electrospun nanofibre material.

12. A use of a nanofibre nonwoven according to one of claims 1 to 8 or produced by a method according to one of claims 9 to 11 for cultivation or differentiation of cells or for non-therapeutic treatment of humans or animals.

13. An artificial tissue comprising a nanofibre nonwoven according to one of claims 1 to 8 or producible by the method of one of claims 9 to 11 and at least one cell of at least one cell type.

14. The artificial tissue according to claim 13, wherein the artificial tissue comprises 1 to 50 wt.% nanofibre nonwoven and 50 to 99 wt.% cells, in particular 80 to 99 wt.%, and, optionally, extracellular matrix (in each case based on the total weight of the artificial tissue), and/or
wherein the at least one porogen (6) present in the nanofibre nonwoven has been at least partially removed from the nanofibre nonwoven prior to colonization with the at least one cell of at least one cell type.

15. A method for producing an artificial tissue according to one of claims 13 to 14, comprising the following method steps:

x1) providing a cell of at least one cell type, a culture medium and at least one nanofibre nonwoven according to any one of claims 1 to 8 or producible by a method according to any one of claims 9 to 11,
x2) optionally removing at least a part of an optionally present at least one porogen (6),
x3) cultivating the at least one nanofibre nonwoven with the at least one cell of the at least one cell type in the culture medium, and
x4) obtaining an artificial tissue comprising at least one cell arranged on or in the at least one nanofibre nonwoven.

16. The method according to claim 15, wherein during or after the cultivation according to method step x3), the nonwoven is cultivated in a method step x31) with at least one cell of at least one further cell type, in particular a cell type different from the cell type provided in method step x1), and/or
wherein during or after the cultivation according to method step x3) or x31), further nanofibre nonwovens, preferably colonized with at least one cell of an identical or different cell type, are stacked on the nanofibre nonwoven obtained in x3) or x31).

17. A nanofibre nonwoven according to any one of claims 1 to 8 or produced by a method of any one of claims 9 to 11 for use in the treatment of diseased humans or animals, in particular for implantation.

**Revendications**

1. Non-tissé de nanofibres, comprenant un réseau de nanofibres (7) formées à partir d'au moins un matériau de nanofibres et entourant des pores (5), fabriqué au moyen d'un procédé comprenant les étapes de procédé :

a) mettre à disposition au moins un matériau de nanofibres et au moins un matériau de porogène approprié à former au moins un porogène,
b) électrofiler l'au moins un matériau de nanofibres en y introduisant l'au moins un porogène (6) formé à partir de l'au moins un matériau de porogène, **caractérisé en ce que** l'au moins un porogène (6) présente un diamètre compris entre 30 et 1000 $\mu$m, faisant en sorte que le rapport volumique entre le matériau de porogène et le matériau de nanofibres soit compris entre 40 à 99 pour 60 à 1 dans le non-tissé de nanofibres électrofilé tel qu'obtenu à l'issue de cette étape de procédé (chacun des volumes étant exprimé par rapport au volume total, donc des fibres et du porogène, du non-tissé de nanofibres), et
c) obtenir un non-tissé de nanofibres comprenant au moins un porogène (6),
lesdites nanofibres se présentant sous forme de couches de nanofibres, un pore étant un espace entouré de couches de nanofibres lequel peut être rempli par des porogènes, des cellules ou des fluides, les pores séparant les différentes couches de nanofibres les unes des autres dans l'espace,
une couche de nanofibres étant une strate constituée de nanofibres superposées et/ou juxtaposées, et
si l'étape de procédé a) implique une mise à disposition du porogène en tant que porogène entièrement formé, l'introduction alternante dudit porogène et l'électrofilage du matériau de nanofibres étant réalisée à l'étape de procédé b), ou
si le matériau de porogène mis à disposition dans l'étape de procédé a) est un matériau approprié à former au moins un porogène sous forme de fibres, au moins un porogène sous forme de fibres étant formé à l'étape de procédé b) à partir dudit matériau de porogène avant ou pendant que celui-ci soit introduit dans le matériau de nanofibres électrofilé.

2. Non-tissé de nanofibres selon la revendication 1, ledit procédé comprenant en outre les étapes de procédé suivantes :

d) incuber le non-tissé de nanofibres, tel qu'obtenu dans l'étape de procédé c) et comprenant au moins un porogène (6), dans au moins un solvant afin de retirer au moins partiellement l'au moins un porogène du non-tissé de nanofibres, et

e) obtenir un non-tissé de nanofibres.

3. Non-tissé de nanofibres selon les revendications 1 ou 2, le matériau de porogène mis à disposition dans l'étape de procédé a) se présentant sous forme de particules ou sous forme de fibres, ayant un diamètre compris entre 30 et 1000 μm et étant mis à disposition dans l'étape de procédé a) en tant que porogène (6) formé.

4. Non-tissé de nanofibres selon les revendications 1 ou 2, le matériau de porogène mis à disposition dans l'étape de procédé a) étant un matériau approprié à former au moins un porogène (6) sous forme de fibres, et l'étape de procédé b) impliquant la formation d'au moins un porogène sous forme de fibres à partir dudit matériau de porogène avant ou pendant que celui-ci soit introduit dans le matériau de nanofibres électrofilé.

5. Non-tissé de nanofibres, comprenant un réseau de nanofibres (7) qui sont constituées d'au moins un matériau de nanofibres et qui entourent des pores (5), la porosité du non-tissé de nanofibres étant comprise entre 90,0 et 99,9 % en volume, notamment entre 98,5 et 99,9 % en volume (toujours par rapport au volume total du non-tissé de nanofibres).

6. Non-tissé de nanofibres selon les revendications 1 à 5, au moins un liquide étant présent dans le non-tissé de nanofibres, notamment de 90,0 à 99,9 % en volume de liquide (par rapport au volume total dudit non-tissé de nanofibres).

7. Non-tissé de nanofibres selon l'une des revendications 1 ou 3 à 6, le non-tissé de nanofibres comprenant en outre des porogènes (6).

8. Non-tissé de nanofibres selon la revendication 7, les porogènes (6) se présentant sous forme de particules ou sous forme de fibres et/ou ayant un diamètre compris entre 30 et 1000 μm.

9. Procédé de fabrication d'un non-tissé de nanofibres comprenant un réseau de nanofibres (7) formées à partir d'au moins un matériau de nanofibres et entourant des pores (5) selon les revendications 1 à 8, comprenant les étapes de procédé suivantes :

a) mettre à disposition au moins un matériau de nanofibres et au moins un matériau de porogène approprié à former au moins un porogène,
b) électrofiler l'au moins un matériau de nanofibres en y introduisant l'au moins un porogène (6) formé à partir de l'au moins un matériau de porogène, **caractérisé en ce que** le porogène (6) présente un diamètre compris entre 30 et 1000 μm, faisant en sorte que le rapport volumique entre le matériau de porogène et le matériau de nanofibres soit compris entre 40 à 99 pour 60 à 1 dans le non-tissé de nanofibres électrofilé tel qu'obtenu à l'issue de cette étape de procédé (chacun des volumes étant exprimé par rapport au volume total, donc des fibres et du porogène, du non-tissé de nanofibres), et
c) obtenir un non-tissé de nanofibres, comprenant au moins un porogène (6), présentant notamment une porosité comprise entre 90,0 et 99,9 % en volume, notamment entre 98,5 et 99,9 % en volume (toujours par rapport au volume total du non-tissé de nanofibres), présentant préférentiellement une porosité comprise entre 90,0 et 99,9 % en volume, notamment entre 98,5 et 99,9 % en volume (toujours par rapport au volume total du non-tissé de nanofibres) et des mailles (8) dont l'ouverture est comprise entre 10 et 200 μm$^2$ (mesurée dans de l'eau sans porogène (6)),
lesdites nanofibres se présentant sous forme de couches de nanofibres, un pore étant un espace entouré de couches de nanofibres lequel peut être rempli par des porogènes, des cellules ou des fluides, les pores ayant pour effet que les différentes couches de nanofibres soient séparés les unes des autres,
une couche de nanofibres étant une strate constituée de nanofibres superposées et/ou juxtaposées, et
si l'étape de procédé a) implique une mise à disposition du porogène en tant que porogène entièrement formé, l'introduction alternant dudit porogène et l'électrofilage du matériau de nanofibres étant réalisée à l'étape de procédé b), ou
si le matériau de porogène mis à disposition dans l'étape de procédé a) est un matériau approprié à former au moins un porogène sous forme de fibres, au moins un porogène sous forme de fibres étant formé à l'étape de procédé b) à partir dudit matériau de porogène avant ou pendant que celui-ci soit introduit dans le matériau de nanofibres électrofilé.

10. Procédé de fabrication d'un non-tissé de nanofibres selon la revendication 9, comprenant en outre les étapes de procédé suivantes :

d) incuber le non-tissé de nanofibres, tel qu'obtenu dans l'étape de procédé c) et comprenant au moins un porogène (6), dans au moins un solvant afin de retirer au moins partiellement l'au moins un porogène du non-tissé de nanofibres, et

e) obtenir un non-tissé de nanofibres.

11. Procédé de fabrication d'un non-tissé de nanofibres selon l'une des revendications 9 ou 10, le matériau de porogène mis à disposition dans l'étape de procédé a) se présentant sous forme de particules ou sous forme de fibres, ayant un diamètre compris entre 30 et 1000 $\mu$m et étant mis à disposition dans l'étape de procédé a) en tant que porogène (6) formé, ou

le matériau de porogène mis à disposition dans l'étape de procédé a) étant un matériau approprié à former au moins un porogène (6) sous forme de fibres, et l'étape de procédé b) impliquant la formation d'au moins un porogène sous forme de fibres à partir dudit matériau de porogène avant ou pendant que celui-ci soit introduit dans le matériau de nanofibres électrofilé.

12. Utilisation d'un non-tissé de nanofibres selon l'une quelconque des revendications 1 à 8 ou fabriqué selon un procédé selon l'une quelconque des revendications 9 à 11 pour cultiver ou différencier des cellules ou pour réaliser un traitement non-thérapeutique d'êtres humains ou d'animaux.

13. Tissu artificiel comprenant un non-tissé de nanofibres selon l'une quelconque des revendications 1 à 8 ou pouvant être fabriqué selon l'une quelconque des revendications 9 à 11, et au moins une cellule d'au moins un type de cellule.

14. Tissu artificiel selon la revendication 13, ledit tissu artificiel comprenant 1 à 50 % en poids de non-tissé de nanofibres et 50 à 99 % en poids de cellules, notamment 80 à 99 % en poids, et optionnellement de la matrice extracellulaire (toujours par rapport au poids total du tissu artificiel) et/ou

l'au moins un porogène (6), présent dans ledit non-tissé de nanofibres, ayant été retiré au moins partiellement du non-tissé de nanofibres, préalablement à la colonisation par l'au moins une cellule d'au moins un type de cellule.

15. Procédé de fabrication d'un tissu artificiel selon l'une des revendications 13 à 14, comprenant les étapes de procédé suivantes :

x1) mettre à disposition au moins une cellule d'au moins un type de cellule, un milieu de culture et au moins un non-tissé de nanofibres selon l'une quelconque des revendications 1 à 8 ou pouvant être fabriqué selon le procédé selon l'une quelconque des revendications 9 à 11,

x2) optionnellement, retirer au moins partiellement l'au moins un porogène (6) si ce dernier est présent,

x3) cultiver l'au moins un non-tissé de nanofibres avec l'au moins une cellule d'au moins un type de cellule dans ledit milieu de culture, et

x4) obtenir un tissu artificiel comprenant au moins une cellule qui est disposée sur ou dans l'au moins un non-tissé de nanofibres.

16. Procédé selon la revendication 15, ledit non-tissé étant cultivé dans une étape de procédé x31), réalisée pendant ou après la mise en culture selon l'étape de procédé x3), avec au moins une cellule d'au moins un autre type de cellule, s'agissant notamment d'un type de cellule qui est différent du type de cellule mis à disposition dans l'étape de procédé x1), et/ou

d'autres non-tissés de nanofibres, qui sont préférentiellement colonisés par au moins une cellule d'au moins un type de cellule identique ou différent, étant superposés au non-tissé de nanofibres obtenu dans x3) ou x31) pendant ou après la mise en culture selon l'étape de procédé x3) ou x31).

17. Non-tissé de nanofibres selon l'une quelconque des revendications 1 à 8 ou pouvant être fabriqué selon un procédé selon l'une quelconque des revendications 9 à 11, destiné à être utilisé pour traiter des êtres humains ou des animaux atteints d'une maladie, notamment à être implanté.

Figur 1

Figur 2

Figur 3

Figur 4

Figur 5

Figur 6

Figur 7

Figur 8

Figur 9

Figur 10

Figur 11

Figur 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2016105581 A1

- WO 2007125288 A1